# EUROPEAN PATENT SPECIFICATION

(11) **EP 2 840 146 B1**
(45) Date of publication and mention of the grant of the patent: **19.07.2017**
(21) Application number: 13730616.3
(22) Date of filing: 19.04.2013
(51) Int. Cl.: C12Q 1/68

(54) **METHOD TO PREDICT THE SAFETY OF THE TREATMENT WITH A NICOTINIC CHOLINERGIC RECEPTOR AGONIST**
VERFAHREN ZUR VORHERSAGE DER SICHERHEIT EINER BEHANDLUNG MIT EINEM AGONISTEN DES NIKOTINISCHEN ACETYLCHOLINREZEPTORS
MÉTHODE DE PRÉDICTION DE LA SÉCURITÉ D'UN TRAITEMENT PHARMACOLOGIQUE

(30) Priority: 20.04.2012 ES 201230590
(43) Date of publication of application: 25.02.2015
(73) Proprietor: Genetracer Biotech S.L., 39011 Santander - Cantabria (ES); Universidad del Pais Vasco, 48940 Leioa Vizcaya (ES)
(72) Inventor: CORTIJO BRINGAS, Carlos, E-39011 Santander - Cantabria (ES); MEANA MARTÍNEZ, José Javier, E-48940 Leioa - Vizcaya (ES); BALLESTEROS RODRÍGUEZ, Francisco Javier, E-48940 Leioa - Vizcaya (ES); GARCÍA-ORAD CARLES, África, E-48940 Leioa - Vizcaya (ES)
(74) Representative: Elzaburu S.L.P.
(86) International application number: PCT/ES2013/070252
(87) International publication number: WO 2013/156657

(56) References cited:
- WO-A2-2011/133949
- ILLUMINA: "Sentrix HumanHap300 Genotyping BeadChip", INTERNET CITATION, 2006, pages 1-4, XP002548936, Retrieved from the Internet: URL:http://www.lerner.ccf.org/services/gc/ documents/HUMANHAP300Datasheet.pdf [retrieved on 2009-10-06]
- AFFYMETRIX: "Data Sheet Affymetrix(R) Genome-Wide Human SNP Array 6.0", INTERNET CITATION, 2007, pages 1-4, XP002525407, Retrieved from the Internet: URL:http://www.affymetrix.com/support/tech nical/datasheets/genomewide_snp6_datasheet .pdf [retrieved on 2009-04-10]
- DAVID P KING ET AL: "Smoking Cessation Pharmacogenetics: Analysis of Varenicline and Bupropion in Placebo-Controlled Clinical Trials", NEUROPSYCHOPHARMACOLOGY, vol. 37, no. 3, 1 February 2012 (2012-02-01), pages 641-650, XP055073703, ISSN: 0893-133X, DOI: 10.1038/npp.2011.232 cited in the application
- SWAN G E ET AL: "Varenicline for smoking cessation: nausea severity and variation in nicotinic receptor genes.", THE PHARMACOGENOMICS JOURNAL AUG 2012, vol. 12, no. 4, 24 May 2011 (2011-05-24), pages 349-358, XP009171677, ISSN: 1473-1150 cited in the application
- ZHANG LAN ET AL: "The [mu]-opioid receptor gene and smoking initiation and nicotine dependence", BEHAVIORAL AND BRAIN FUNCTIONS, BIOMED CENTRAL, LONDON, GB, vol. 2, no. 1, 4 August 2006 (2006-08-04), page 28, XP021019856, ISSN: 1744-9081, DOI: 10.1186/1744-9081-2-28
- RAY R ET AL: "Association of OPRM1 A118G variant with the relative reinforcing value of nicotine", PSYCHOPHARMACOLOGY, SPRINGER, BERLIN, DE, vol. 188, no. 3, 8 September 2006 (2006-09-08), pages 355-363, XP019420113, ISSN: 1432-2072, DOI: 10.1007/S00213-006-0504-2
- M. QUAAK ET AL: "Genetic variation as a predictor of smoking cessation success. A promising preventive and intervention tool for chronic respiratory diseases?", EUROPEAN RESPIRATORY JOURNAL, vol. 33, no. 3, 1 March 2009 (2009-03-01), pages 468-480, XP055073854, ISSN: 0903-1936, DOI: 10.1183/09031936.00056908
- ELENA V. RADCHENKO ET AL: "Agonist and antagonist effects of cytisine in vivo", NEUROPHARMACOLOGY., vol. 95, 31 March 2015 (2015-03-31), pages 206-214, XP055242854, GB ISSN: 0028-3908, DOI: 10.1016/j.neuropharm.2015.03.019
- NATALIE WALKER ET AL: "Cytisine versus Nicotine for Smoking Cessation", NEW ENGLAND JOURNAL OF MEDICINE, vol. 371, no. 25, 18 December 2014 (2014-12-18), pages 2353-2362, XP055242858, US ISSN: 0028-4793, DOI: 10.1056/NEJMoa1407764
- SERENA TONSTAD ET AL: "Dianicline, a Novel [alpha]4[beta]2 Nicotinic Acetylcholine Receptor Partial Agonist, for Smoking Cessation: A Randomized Placebo-Controlled Clinical Trial", NICOTINE & TOBACCO RESEARCH, vol. 13, no. 1, 1 November 2010 (2010-11-01), pages 1-6, XP055242862,

## Description

### Field of the Invention

The present invention generally relates to methods for predicting the safety of a nicotinic cholinergic receptor agonist drug-based pharmacological treatment based on the presence of certain polymorphisms.

### Background of the Invention

Varenicline is a derivative compound of cytisine which acts as a selective partial nicotinic cholinergic receptor agonist which binds with high affinity and selectivity to the α4β2 neuronal nicotinic acetylcholine receptors subtype.

Varenicline is used in the treatment of various diseases, among which there are included chronic inflammatory bowel diseases (such as ulcerative colitis, pyoderma gangrenosum and Crohn's disease), irritable bowel syndrome, spasmodic dystonia, chronic pain, acute pain, celiac disease, pouchitis, vasoconstriction, anxiety, panic disorder, depression, bipolar disorder, autism, sleep disorder, jet lag, amyotrophic lateral sclerosis (ALS), cognitive dysfunction, hypertension, bulimia, anorexia, obesity, heart arrhythmia, gastric acid hypersecretion, ulcer, pheochromocytoma, progressive supranuclear palsy, chemical addiction and dependence (nicotine, tobacco products, alcohol, benzodiazepine, barbiturates, opioids, cocaine), headache, migraine, stroke, traumatic brain injury (TBI), obsessive-compulsive disorder, psychosis, Huntington's disease, tardive dyskinesia, hyperkinesia, dyslexia, schizophrenia, multi-infarct dementia, age-associated cognitive impairment, epilepsy, Alzheimer's disease senile dementia, Parkinson's disease, attention deficit-hyperactivity disorder (ADHD) and Tourette syndrome.

One of the most widespread uses of varenicline is the use thereof in pharmacological treatment intended for quitting tobacco consumption.

Pharmacological treatment with varenicline is known to lead to various adverse effects (Leung LK et al. 2011 BMC Clin Pharmacol 11:15; Ebbert O et al. 2010 Patience Prefer Adherence 4:355-362), among which the following have been described: nausea (the most common adverse effect), headache, vomiting, flatulence, insomnia, abnormal dreams and alteration of sense of taste (dysgeusia). Neuropsychic disorders associated with the treatment with varenicline, such as mood swings, irritability, drowsiness, poor physical coordination, hallucinations, paranoid reactions, depression and suicidal behaviors, have also been described.

The United States Food and Drug Administration (FDA) has issued various public health recommendations relating to varenicline-based treatment. In the year 2008, the FDA requested for the inclusion of a warning in varenicline-based medicinal products regarding the possibility of mood swings and behavioral changes in patients under treatment with that drug. In the year 2009, the FDA requested for the inclusion of a warning regarding the possibility of mood swings such as hostility, agitation, depression and suicidal ideation or actions. The safety communication issued by the FDA on 2011 concerning varenicline-based medicinal products maintains the warning concerning possible neuropsychic events and additionally indicates that smoking cessation treatments based on said products entail a small increase in the incidence of adverse cardiovascular events in patients with stable cardiovascular disease.

The onset of adverse effects in the subject under pharmacological treatment is a common cause for quitting said treatment. The cessation of the pharmacological treatment before the end thereof results immediately in partial or complete loss of effectiveness since not all the benefits which the medicinal products can provide for the patients are obtained, and furthermore entails an increase in health expenditure. In developed countries, the therapeutic adherence (part of the human behavior involved in health and expressing the responsibility of the individuals with respect to health care and maintenance) in patients suffering chronic diseases is only about 50%, i.e., only half the patients with chronic suffering correctly perform the indicated treatment necessary for controlling their disease, which jeopardizes the capability of the health system to achieve the objectives relating to the population's health.

Pharmacogenetic analyses performed in populations of smokers under pharmacological smoking cessation treatment with varenicline show a relationship between the presence of certain polymorphisms in genes relating to the medicinal product metabolism, nicotinic receptors, or varenicline transporters, among others, and the development of nausea as a result of said varenicline-based treatment (King DP et al. 2012 Neuropsychopharmacol 37:641-650; Swan GE et al. 2011 Pharmacogenomics J doi:10.1038/tpj.2011.19; US2009176878A1). However, the genes relating to nicotinic receptors described in such studies refer to a pharmacological target of varenicline which is not extensible to the neurobiological processes of dependence.

Despite the fact that varenicline is used in the treatment of various diseases, studies which allow determining the safety of varenicline-based pharmacological treatments, or which allow identifying subjects for whom a varenicline-based pharmacological treatment is suitable, have not been performed up until now. Therefore, it is necessary to develop methodologies which allow, on one hand, determining the probability that a subject will present adverse effects in response to a pharmacological treatment with varenicline, or another drug with a similar mechanism of action, such as another nicotinic cholinergic receptor agonist drug, for example, cytisine, dianicline, etc., that lead to the withdrawal of said treatment or to a reduction of the usual therapeutic dose of the drug used in said pharmacological treatment, and, on the other hand, identifying subjects for whom the varenicline- or other nicotinic cholinergic receptor agonist drug-based pharmacological treatment is safe.

### Brief Description of the Invention

The authors of the present invention have identified single-nucleotide polymorphisms (SNPs) the presence of which is related to the safety of a varenicline- (or other nicotinic cholinergic receptor agonist drug) based pharmacological treatment. The inventors have conducted a study to determine the pharmacogenomic profile of smoker subjects subjected to smoking cessation treatment with varenicline associated with the safety of said pharmacological treatment and have evaluated the results obtained in terms of dose reduction or of pharmacological treatment suspension due to the onset of adverse effects. Briefly, 384 SNPs were genotyped in a total of 807 subjects, 479 of whom had received or were receiving pharmacological treatment with varenicline, and the association of the genotypes with the onset of adverse effects in response to said drug was studied, the alleles of certain SNPs, specifically of SNPs rs7930792, rs12423809, rs4251417, rs7146, rs477292, rs495491, rs3778151, rs9479757, rs763132, rs4474069 or rs1183035, being found to be significantly associated. SNP rs7930792 is located in the *UBASH3B* (*ubiquitin associated and SH3 domain containing B)* gene, SNP rs12423809 is located in the *C12orf36* (*chromosome 12 open reading frame 36)* gene, SNP rs4251417 is located in the *SLC6A4* (*solute carrier family 6 member 4)* gene, SNP rs7146 is located in the *C19orf6 (chromosome 19 open reading frame 6)* gene, SNPs rs477292, rs495491, rs3778151 and rs9479757 are located in the *OPRM1 (opioid receptor mu 1*) gene, SNP rs763132 is located in the *ARHGAP18 (Rho GTPase activating protein 18*) gene, SNP rs4474069 is located in the *RAPGEF1* (*Rap guanine nucleotide exchange factor 1*) gene and SNP rs1183035 is located in the *MAOB* (*monoamine oxidase B*) gene. This finding opens the door to new genetic predictors useful in predicting the onset of adverse effects in response to a pharmacological treatment based on varenicline or on drugs with a similar mechanism of action (nicotinic cholinergic receptor agonists), such as for example, cytisine and dianicline, among others. The inventors have developed the methods of the present invention based on these findings. The information provided by said methods can be efficiently used by the specialist in designing customized medicine intended for the treatment of pathologies that can be treated with nicotinic cholinergic receptor agonist drugs, for example, for the treatment of tobacco addiction.

The inventive aspects of the present invention are mentioned in the claims.

In a particular embodiment applicable to most inventive aspects of the invention object of the present patent application [method for determining the probability that a subject will suffer adverse effects in response to a nicotinic cholinergic receptor agonist drug-based therapy, method for predicting the safety of a nicotinic cholinergic receptor agonist drug-based therapy, method for selecting a subject to follow a nicotinic cholinergic receptor agonist drug-based therapy, method for selecting a therapy, or use of SNPs according to the invention], said SNP is an SNP located in the *OPRM1* gene selected from the group consisting of SNPs rs477292, rs495491, rs3778151, rs9479757 and any combination of said SNPs. Besides, a kit consisting of the reagents (allele-specific or polymorphism-specific oligonucleotide pairs or a set of oligonucleotide probes targeting each of the SNPs) for determining the presence of at least one allele or the nucleotide present in the polymorphic site of each of the mentioned SNPs and the use of said kit for performing the objectives of the above mentioned methods are also aspects of the present invention.

In another particular embodiment applicable to the methods and the use of SNPs which are inventive aspects of the invention object of the present patent application, said SNP is an SNP of a gene located in the human chromosome 6 selected from the group consisting of SNPs rs477292, rs495491, rs3778151, rs9479757 and rs763132 and any combination of said SNPs

In another particular embodiment applicable to the methods and the use of SNPs which are inventive aspects of the invention object of the present patent application, said SNP is an SNP of a gene involved in the cyclic AMP (cAMP)-mediated signaling pathway selected from the group consisting of SNPs rs477292, rs495491, rs3778151, rs9479757, rs4474069 and any combination of said SNPs. The SNPs rs477292, rs495491, rs3778151, rs9479757 are located in the *OPRM1* gene whereas the SNP rs4474069 is located in the *RAPGEF1* gene; both genes *(OPRM1* and *RAPGEF1*) are involved in the cAMP-mediated signaling pathway.

In another particular and preferred embodiment applicable to the methods and the use of SNPs which are inventive aspects of the invention object of the present patent application, said SNP is the SNP rs9479757; this SNP can be used alone for predicting the safety of a nicotinic cholinergic receptor agonist drug-based therapy.

### Brief Description of the Drawings

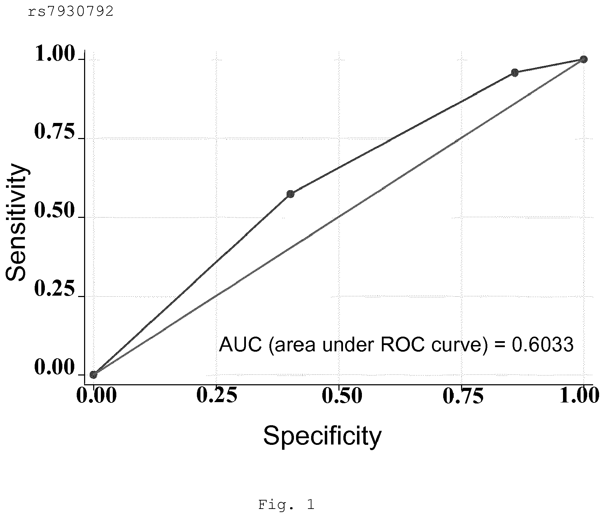

Figure 1 shows the ROC curves corresponding to the univariate models in relation to SNP rs7930792.

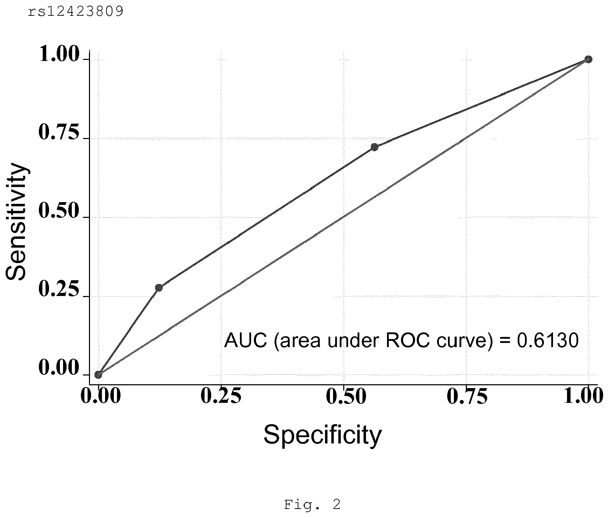

Figure 2 shows the ROC curves corresponding to the univariate models in relation to SNP rs12423809.

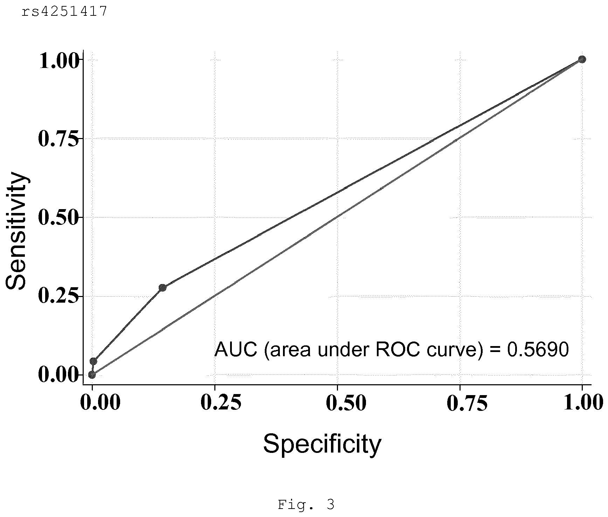

Figure 3 shows the ROC curves corresponding to the univariate models in relation to SNP rs4251417.

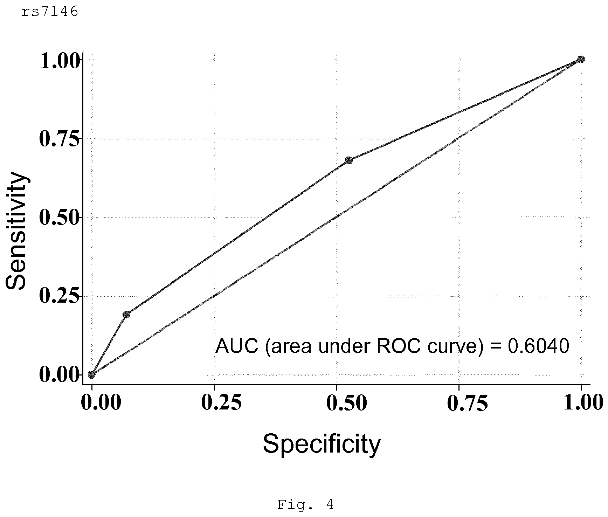

Figure 4 shows the ROC curves corresponding to the univariate models in relation to SNP rs7146.

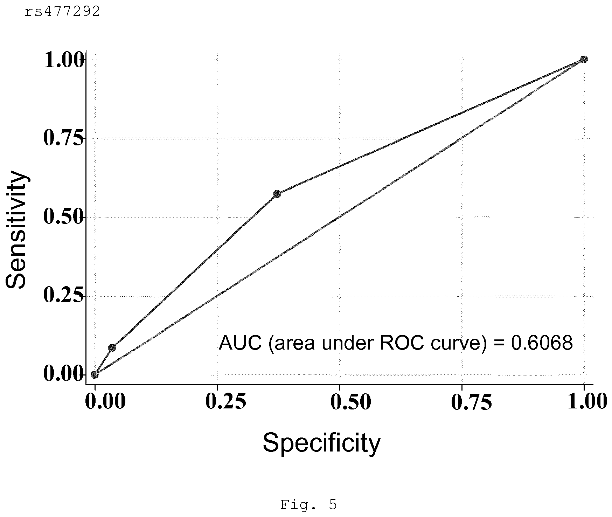

Figure 5 shows the ROC curves corresponding to the univariate models in relation to SNP rs477292.

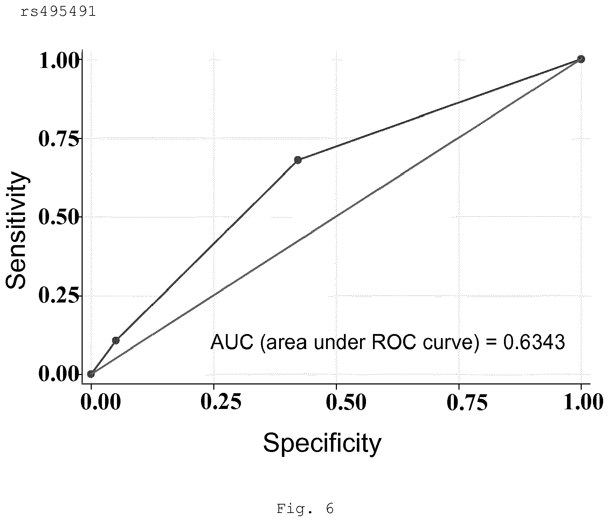

Figure 6 shows the ROC curves corresponding to the univariate models in relation to SNP rs495491.

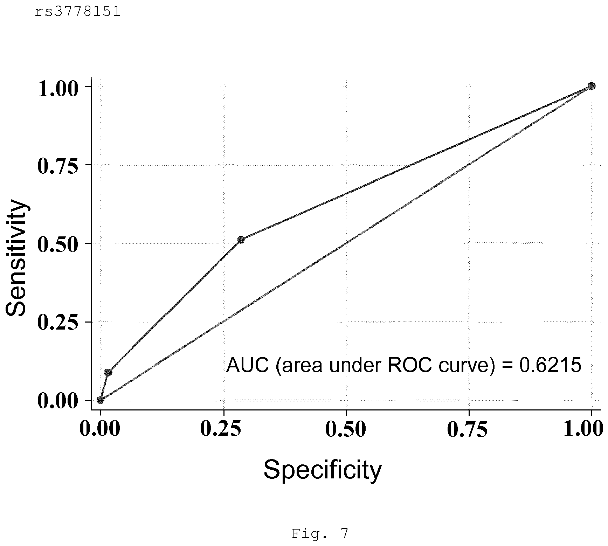

Figure 7 shows the ROC curves corresponding to the univariate models in relation to SNP rs3778151.

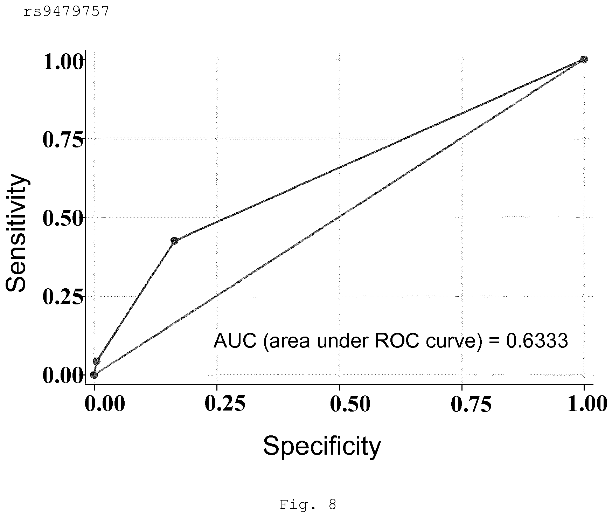

Figure 8 shows the ROC curves corresponding to the univariate models in relation to SNP rs9479757.

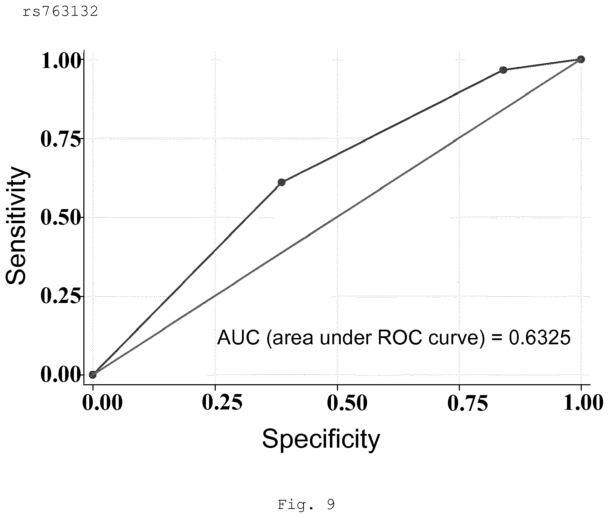

Figure 9 shows the ROC curves corresponding to the univariate models in relation to SNP rs763132.

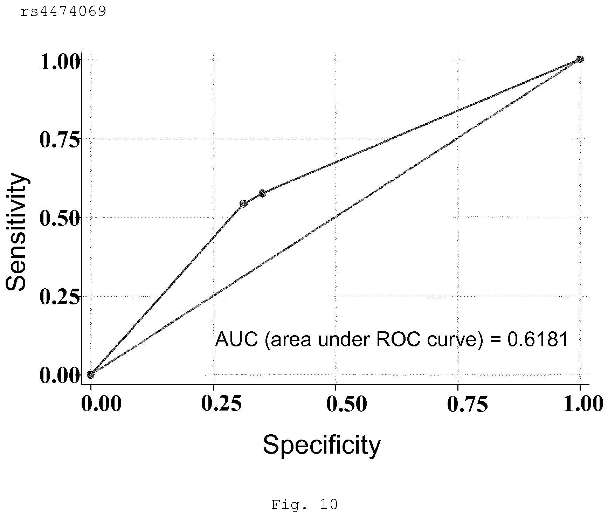

Figure 10 shows the ROC curves corresponding to the univariate models in relation to SNP rs4474069.

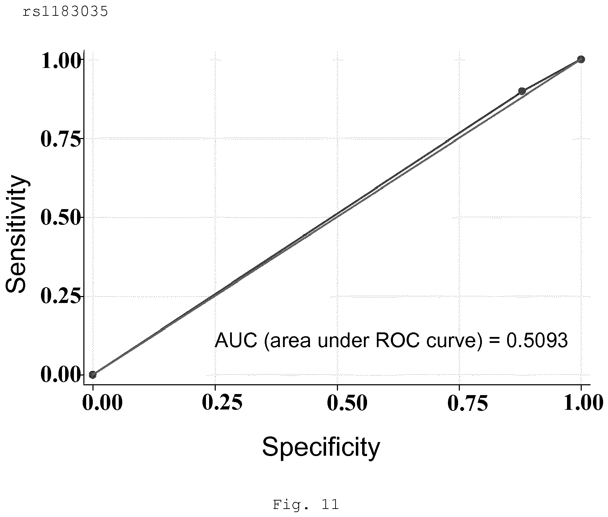

Figure 11 shows the ROC curves corresponding to the univariate models in relation to SNP rs1183035.

### Detailed Description of the Invention

The authors of the present invention have identified alleles of SNPs the presence of which is related to a higher or lower probability of the onset of adverse effects in response to a pharmacological treatment based on varenicline or on drugs with a similar mechanism of action, such as for example, cytisine and dianicline, among others. The methods of the present invention which are described in detail below have been developed based on that finding.

Tobacco consumption is a complex disease involving genes associated with the start and the progression in consumption, the dependence and the cessation of tobacco consumption. The SNPs identified by the authors of the present invention are located in genes involved in biologically connected metabolic pathways involved in the pharmacogenetics of tobacco consumption. In this manner, SNP rs7930792 is located in the *UBASH3B* gene relating to the ubiquitination pathway; SNP rs4251417 is located in the *SLC6A4* gene relating to serotonin neurotransmission; SNP rs7146 is located in the *c19orf6* gene relating to glutamate receptor signaling; SNP rs1183035 is located in the *MAOB* gene relating to dopamine neurotransmission and metabolic synthesis; SNP rs763132 is located in the *ARHGAP18* gene relating to tight junction signaling; SNPs rs477292, rs495491, rs3778151 and rs9479757 are located in the *OPRM1* gene relating to cAMP-mediated signaling; SNP rs4474069 is located in the *RAPGEF1* gene also relating to cAMP-mediated signaling.

To avoid any doubts, the methods of the invention are carried out with a sample which is previously withdrawn from the subject. The kits of the invention described below can include means for extracting the sample from the subject.

### Definitions

The following definitions are presented to illustrate and define the meaning and scope of different terms and expressions used for describing the invention itself.

In the present invention, the terms "quit" or "cessation" are used interchangeably in relation to tobacco consumption to refer to the abstinence from, the termination or end of tobacco consumption. Quitting tobacco consumption can be (i) complete when there is a total absence of consumption of any type or form of tobacco product from the time in which a decision is made to quit the consumption thereof, (ii) sustained when the subject who quits consuming tobacco has sustained or punctual relapses for 15 days after the date on which he/she decides to cease or quit consuming tobacco, or (iii) temporary when the subject quits consuming tobacco for a variable period of time albeit a short one, generally of weeks or months. Advantageously, the cessation of tobacco consumption is a complete cessation. In clinical practice, verbal declaration for the cessation of tobacco consumption, when the tobacco is inhaled (smoked), can be validated by means of the determination of carbon monoxide (CO) levels in exhaled air, co-oximetry being the most common technique for said determination. Levels of 10 ppm or more of CO in the exhaled air correspond to smoker subjects. Levels of 6 to 10 ppm correspond to occasional smokers, and figures below 6 ppm correspond to non-smokers.

As used herein, the expression "tobacco addiction" or "tobacco dependence" refers to the abuse of tobacco consumption, causing the tobacco consumer to suffer physical and psychological dependence generating an abstinence syndrome. Tobacco is addictive mainly due to nicotine which also has antidepressant effects and provides symptomatic relief of anxiety. The term "tobaccoism" is sometimes used as a synonym of "tobacco addiction"; nevertheless, the term "tobaccoism" is also used in relation to a chronic intoxication which occurs due to tobacco abuse and includes the effects of repeated tobacco consumption and of the developed addiction. Tobacco addiction is a risk factor of respiratory and cardiovascular diseases and is particularly harmful during pregnancy. Tobaccoism is related to the onset of a high number of diseases including several types of cancer, lung cancer being the most common. Furthermore, tobacco does not only harm active smokers, but also those who are breathing in the same air (passive smokers).

The term "agonist" refers to a compound which is capable of generating a response in a cell after binding to a cell receptor. The agonist compound binds to a receptor and has an intrinsic effect and therefore increases the basal activity of a receptor when it contacts the receptor. On the other hand, the term "antagonist" refers to a compound which, upon binding to a cell receptor, blocks the activation of said cell receptor by the agonist. The antagonist compound competes with the agonist compound to bind to a receptor, thus blocking the action of the agonist in the receptor. However, an antagonist (also known as "neutral" antagonist) does not have any effect on the constitutive activity of the receptor. Antagonists mediate their effects by means of binding to the active site or to the allosteric sites in receptors, or they may interact at unique binding sites not usually involved in the biological regulation of the receptor's activity. The antagonist activity can be reversible or irreversible depending on the longevity of the antagonist-receptor complex which in turn depends on the nature of the antagonist-receptor binding. Likewise, the agonists can be partial or complete. A "partial agonist" is defined as a compound having affinity for a receptor, but unlike a complete agonist, it will only cause a small degree of pharmacological response characteristic of the nature of the receptor involved, even if a high proportion of receptors are occupied by the compound.

As used herein, the term "allele" refers to one, two or more forms of a gene, locus or genetic polymorphism. The different alleles can sometimes give rise to different phenotypes; however, other times, the different alleles will have the same result in gene expression. Most multicellular organisms have two sets of chromosomes, i.e., they are diploids. These chromosomes are called homologous chromosomes. Diploid organisms have a copy of each gene (and one allele) in each chromosome. If both alleles are the same, they are homozygotes. If the alleles are different, they are heterozygotes.

As used herein, the term "linkage group" or "haplotypic group" refers to the set of SNPs in a particular chromosome that are statistically associated and configure an haplotype (combination of genetic markers).

As used herein, the term "cytisine" refers to the compound (IP,5S)-1,2,3,4,5,6-hexahydro-1,5-methane-8H-pyrido[1,2a][1,5]diazocin-8-one, of formula:

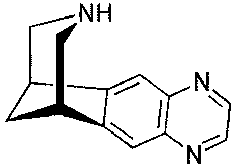

Cytisine is a plant alkaloid having a relatively rigid conformation with a molecular structure similar to that of nicotine and acetylcholine. Cytisine is a natural insecticide found in the leaves of *Cytisus laburnum* and *Ulex europaeus,* among others. The synthesis of cytisine and derivates has been described in Coe JW 2000 Org Lett 2:4205-4208 and O'Neill BT et al. 2000 Org Lett 2:4201-4204. As used herein, the term "cytisine" includes the derivatives mentioned in said scientific publications, among others.

Cytisine is marketed under the brand Tabex^{®} and is comprised within the group of nicotinic cholinergic receptor agonist drugs and is a compound having affinity for the α4β2 neuronal nicotinic acetylcholine receptor subtype. Cytisine is a natural insecticide found in the leaves of *Cytisus laburnum* and *Ulex europaeus,* among others. The synthesis of cytisine and derivatives is described in Coe JW 2000 Org Lett 2:4205-4208 and O'Neill BT et al. 2000 Org Lett 2:4201-4204.

As used herein, the expression "tobacco consumption" includes any form of tobacco consumption, inhaling the products from the burning of tobacco constituting the most common way of consumption. In the sense used in this description, the term "smoking" refers to the action of inhaling and blowing out tobacco smoke. Two types of smoke streams are produced when consuming a cigarette: (i) the first stream or mainstream which is the stream that goes through the inside of the cigarette until reaching the lungs of the active smoker when he/she takes a puff; and (ii) the second stream or sidestream which is the stream that is released to the environment from the lit end of the cigarette and can be inhaled by a subject breathing in that contaminated environment, said subject being the passive smoker. By inhaling tobacco smoke, the average smoker consumes between 1 and 2 mg of nicotine per cigarette. When tobacco is smoked, nicotine quickly reaches maximum levels in the bloodstream and penetrates the brain.

Even though smoking cigarettes is the most common and popular form of tobacco consumption, in the present invention other forms of smokeless tobacco consumption are also contemplated, such as powdered tobacco or snuff consumption and chewing tobacco consumption, for example, that involve forms of tobacco consumption by means of chewing, sucking, sniffing, etc. These smokeless tobacco products also contain nicotine. In the case where the smoke is not inhaled, for example, when smoking pipes or cigars or when consuming smokeless tobacco, nicotine is absorbed through the mucous membranes and reaches the maximum levels in blood and in brain more slowly.

Another form of tobacco consumption, where the prediction of success or failure of consumption cessation in subjects subjected to treatment with varenicline also falls within the objectives of the methods provided by the present invention, is the electronic cigarettes. "Electronic cigarettes" ("e-Cig") are apparatuses vaporizing the substance contained in cartridges, causing the expulsion of vapor that imitates the smoke of the conventional cigarette and achieving a similar effect in the user. The nozzle of the apparatus contains an exchangeable or rechargeable liquid-filled cartridge. The main substances of said liquid are propylene glycol and/or vegetable glycerin, optionally nicotine in different doses (generally between 0 and 36 mg/ml), optional flavors and aromas.

The term "dianicline" refers to the compound (5aS,8S,10aR)-5a,6,9,10-tetrahydro,7H,11H-8,10a-methanepyrido[2',3':5,6]pyrano[2,3-d]azepine having the following formula:

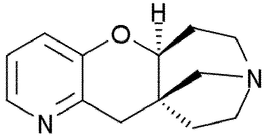

Dianicline is comprised within the group of nicotinic cholinergic receptor agonist drugs and is a compound having affinity for the α4β2 neuronal nicotinic acetylcholine receptor subtype.

In the context of the present invention, the expression "usual therapeutic dose" of a drug refers to the usually administered therapeutic dose of a drug, in this case, a nicotinic cholinergic receptor agonist drug. The therapeutic dose of a drug is the dose (or amount of drug which is administered to effectively achieve a certain effect) comprised between the minimum dose (a small dose and point in which the drug starts to produce an evident pharmacological effect) and the maximum dose (the highest amount of drug that can be tolerated by a subject without causing toxic effects).

In the case of a varenicline-based therapy, the usual therapeutic dose to stop smoking is the following:
- week 1: 0.5 milligrams (mg) once a day for days 1 to 3 of treatment and 0.5 mg twice a day for days 4 to 7 of treatment;
- week 2: 1 mg twice a day for days 8 to 14 of treatment;
- weeks 3 to 12: 1 mg twice a day starting from day 15 of treatment and to the end of treatment.

Likewise, the usual therapeutic dose of varenicline for reducing or quitting alcohol consumption by individuals who consume alcohol regularly, for example, an average of at least 15 alcoholic drinks (12 g ethanol/drink) per week or men who consume an average of at least 20 alcoholic drinks (12 g ethanol/drink) per week, or for treating alcoholism or alcohol dependence, is equal or similar to the usual therapeutic dose of varenicline mentioned above in relation to the use thereof to stop smoking [http://clinicalstudies.info.nih.gov/cgi/detail.cgi?A_2008-AA-0137.html; http://clinicaltrials.gov/ct2/results?term=Varenicline+to+Redu ce+Alcohol+Consumption].

In the case of a cytisine-based therapy, the usual therapeutic dose to stop smoking is the following [http://www.tabex.net/]:
- first 3 days: 1.5 mg, six times a day (every 2 hours), accompanied by a parallel reduction in the number of smoked cigarettes [if the result at the end of the first 3 days of treatment is unsatisfactory, the treatment is suspended; if it is satisfactory, the usual treatment follows the protocol indicated below];
- from day 4 to day 12: 1.5 mg, five times a day (every 2.5 hours);
- from day 13 to day 16: 1.5 mg, four times a day (every 3 hours);
- from day 17 to day 20: 1.5 mg, three times a day (every 5 hours); and
- from day 21 to day 25: 1.5 mg, once or twice a day.

In the case of a dianicline-based therapy, the usual therapeutic dose to stop smoking can be 40 mg, twice a day [http://clinicaltrials.gov/show/NCT00356967; https://www.clinicaltrialsregister.eu/ctr-search/search?query=eudract_number:2006-001099-20].

As used herein, the term "adverse effects" generally refers to the adverse consequences of using a medicinal product or a therapy, and includes the unwanted anticipated symptoms that the subjects can have in response to the prescription of a certain medicinal product or therapy; adverse effects can be produced with the same doses at which therapeutic effect is produced, or at unsuitable or incorrect doses. In this sense, the term "adverse effect" is equivalent to an adverse drug reaction (ADR), defined as "any unintended, harmful reaction (or response to a medicinal product) that occurs at doses normally used in human being for prophylaxis, diagnosis or treatment or for modifying physiological functions" (WHO Chronicle, 1973, 27:476-480). ADR can generally be classified as predictable reactions which represent about 80% of the adverse effects, are dose-dependent and related to pharmacological actions of the medicinal product (overdose, collateral effect, side effect and interaction with other drugs), and as unpredictable reactions which are non dose-dependent, not related to the pharmacological actions of the medicinal product and are linked to patient-dependent factors, since they usually occur in subjects with immune sensitivity or susceptible subjects due to genetic differences. Adverse effects derived from a pharmacological treatment can be "very common" when they can affect at least 1 subject of every 10 subjects; "common" when they can affect at least 1 subject of every 100 subjects; "uncommon" when they can affect at least 1 subject of every 1,000 subjects; and "rare" when they can affect at least 1 subject of every 1,000 subjects.

During the development prior to the marketing of varenicline, more than 4,500 subjects were exposed to varenicline and of those, more than 450 received treatment for at least 24 weeks, and about 100 of said subjects received treatment for 1 year. Most of the study participants were treated for 12 weeks or less. In the placebo-controlled phase II and III studies, the treatment withdrawal rate due to adverse effects in patients treated with 1 mg of varenicline twice a day was 12% (subjects treated with varenicline) and 10% (subjects treated with placebo) in the 3-month treatment studies. In this group, the withdrawal rate due to the most common adverse effects among patients treated with varenicline were the following: nausea (3%) [0.5% in patients treated with placebo]; headache (0.6%) [0.9% in patients treated with placebo]; insomnia (1.2%) [1.1% in patients treated with placebo]; and sleep alterations (0.3%) [0.2% in patients treated with placebo]. The adverse events were categorized according to the Medical Dictionary for Regulatory Activities, MedDRA, version 7.1.

The summary of product characteristics for the medicinal product Champix®, which contains varenicline as an active ingredient, published by the Spanish Agency of Medical Products and Medical Devices, includes a list of adverse effects emerging from the treatment reported by the patients treated with varenicline during the clinical studies; the following are indicated among said adverse effects:
Hematological and lymphatic system disorders: Uncommon: anemia, lymphadenopathy. Rare: leukocytosis, thrombocytopenia, splenomegaly.
Heart disorders: Uncommon: angina pectoris, arrhythmia, bradycardia, ventricular extrasystoles, myocardium infarction, palpitations, tachycardia. Rare: auricular fibrillation, heart flatter, coronary artery disease, pulmonary heart disease, acute coronary syndrome. Disorder of the inner ear and of the labyrinth: Uncommon: tinnitus, vertigo. Rare: hearing loss, Meniere's disease.
Endocrine disorders: Uncommon: thyroid disorders.
Eye disorders: Uncommon: conjunctivitis, dry eyes, eye irritation, blurry vision, visual disturbances, pain in the eye. Rare: acquired nocturnal amblyopia, temporary blindness, subcapsular cataract, vascular eye disorders, photophobia, floaters.
Gastrointestinal disorders: Common: diarrhea, gingivitis. Uncommon: dysphagia, enterocolitis, belching, gastritis, gastrointestinal bleeding, mouth ulcers, esophagitis. Rare: stomach ulcer, bowel obstruction, acute pancreatitis.
General disorders and involvement in the administration site: Common: chest pain, flu-like syndrome, edema, thirst. Uncommon: chest discomfort, shivers, pyrexia. Hepatobiliary disorders: Uncommon: gallbladder disorders.
Immune system disorders: Uncommon: hypersensitivity. Rare: drug hypersensitivity.
Examinations: Common: abnormalities in hepatogram, weight gain. Uncommon: electrocardiographic abnormalities, increase in muscle enzymes, abnormalities urine analyses.
Metabolic and nutritional disorders: Uncommon: diabetes mellitus, hyperlipidemia, hypokalemia. Rare: hyperkalemia, hypoglycemia.
Musculoskeletal and connective tissue disorders: Common: arthralgia, backache, muscle cramps, musculoskeletal pain, myalgia. Uncommon: arthritis, osteoporosis. Rare: myositis.
Nervous system disorders: Common: alterations in attention, dizziness, sensory alterations. Uncommon: amnesia, migraine, parosmia, motor hyperactivity, restless legs syndrome, tremors. Rare: altered balanced, stroke, convulsion, dysarthria, facial paralysis, mental decline, multiple sclerosis, nystagmus, declined psychomotor skills, transient ischemic attack, visual field defect.
Psychiatric disorders: Common: anxiety, depression, emotional disturbances, irritability, restlessness. Uncommon: aggression, agitation, disorientation, dissociation, reduced libido, mood swings, abnormal thoughts. Rare: bradyphrenia, euphoria, hallucinations, psychotic disorders, suicidal ideation.
Kidney and urinary disorders: Common: polyuria. Uncommon: kidney stones, nocturia, urinary abnormalities, urethral syndrome. Rare: acute kidney failure, urinary retention.
Reproductive system and mammary gland disorders: Common: menstrual disorders. Uncommon: erectile dysfunction. Rare: sexual dysfunction.
Respiratory, chest wall and mediastinal disorders: Common: epistaxis, respiratory disorders. Uncommon: asthma. Rare: pleurisy, pulmonary embolism.
Cutaneous and subcutaneous tissue disorders: Common: hyperhidrosis. Uncommon: acne, dermatitis, dry skin, eczema, erythema, psoriasis, urticaria. Rare: photosensitivity reaction.
Vascular disorders: Common: hot flashes, hypertension. Uncommon: hypotension, peripheral ischemia, thrombosis.

The following have been described in relation to the varenicline-based pharmacological treatment:
- very common adverse effects such as headache, difficulty sleeping, abnormal dreams and nausea;
- common adverse effects such as increased appetite, changes in the sense of taste, dry mouth, sleepiness, fatigue, dizziness, vomiting, constipation, diarrhea, feeling bloated, stomach discomforts, indigestion and flatulence;
- uncommon adverse effects such as bronchial infection, discomfort or pain, sinusitis, fever, feeling cold, feeling weak or discomfort, viral infection, shortness of breath, cough, hoarseness, sore and itchy throat, stuffy nose sinuses, runny nose, snoring, loss of appetite, feeling thirsty, weight gain, depression, anxiety, hallucinations, feeling panic, difficulty thinking, mood swings, tremors, difficulty in coordination, difficulty speaking, less touch sensitivity, increased muscle tension, restlessness, altered heart rate, increased blood pressure, increased heart rate, altered vision, eye ball discoloration, pain in the eye, dilated pupils, myopia, sensitivity to light, watery eyes, ringing in the ears, bloody vomit, irritated and acidic stomach, abdominal pain, abnormal stools, red blood in stools, belching, mouth ulcers, gum pain, blackened tongue, skin rash, cysts, fungal infection, reddening of the skin, itching, acne, increased sweating, chest wall and rib pain, numb joints, muscle spasms, glucose in urine, increased urination frequency and volume, increased menstrual flow, vaginal secretion and changes in sex drive and ability; and
- rare adverse effects such as ictus.

Other adverse effects related to the pharmacological treatment with varenicline the frequency of which is unknown include heart attack, changes in the way of thinking or in behavior (such as aggressive and abnormal behavior), suicidal ideation, serious allergic reactions including angioedema (face, mouth or throat swelling) and serious skin reactions including erythema multiforme and Stevens-Johnson syndrome (blisters on skin, mouth and around the eyes and genitals).

The main adverse effects depending on the frequency of their onset and which can lead to the withdrawal of the varenicline- (or other nicotinic cholinergic receptor agonist drug)-based therapy or to a reduction of the usual therapeutic dose of the drug used in said therapy are selected from the group consisting of insomnia, nocturnal agitation, drowsiness, vivid dreams, headaches, dizziness, restlessness, nervous tension, symptoms of depression, bloated feeling in the stomach, abdominal distension, meteorism, flatulence, nausea, vomiting, diarrhea, constipation, dry mouth, aphthae, skin rash, fatigue, general aches and pains and combinations thereof.

The term "nicotinic cholinergic receptor agonist drug" is understood as that drug capable of modifying cell response processes and generating a biological response after interacting with any of the subunits of the nicotinic cholinergic receptor (Florez et al., Farmacologia Humana, 4th Edition, Masson, Barcelona, 2003). Non-limiting illustrative examples of said drugs include cytisine, dianicline and varenicline, among others.

As used herein, the term "biological sample" includes any biological material which can be obtained from a subject, which can be preserved and which can store information about the characteristic genetic makeup of the subject. The sample of the invention can be cell, tissue or fluid type. In a particular embodiment of the invention, the sample is a blood and/or saliva sample.

As used herein, the term "nicotine" refers to an alkaloid compound found in tobacco plant which is responsible for the psychoactive and addictive effects of tobacco. Although close to 5,000 chemical compounds in different phases (gas, solid or particles) have been identified from tobacco smoke, the nicotine content thereof is what makes tobacco addictive. Nicotine is considered to be responsible both for tobacco addiction and for maintaining smoking habits. By way of example, when tobacco smoke is inhaled, nicotine is rapidly absorbed both in the oral mucosa and in the lungs, from where it goes through the circulatory system and is distributed throughout the entire body, and in 7-10 seconds, the nicotine reaches the brain where it binds to the nicotinic receptors producing a pleasant and gratifying stimulant and/or sedative effect triggering the onset of tobacco dependence (pharmacological or physical dependence). The smoker generally adapts his/her nicotine inhalation pattern in terms of frequency, depth of inhalations and smoke retention time, depending on his/her own characteristics and on the effect to be achieved in each circumstance. These variations in the smoking pattern produce different blood nicotine concentrations yielding different levels of nicotine supply to the body and different effects in the action thereof. When the smoker stops smoking for some time, he/she starts to have a series of symptoms caused by the abstinence syndrome. In addition to this pharmacological or physical dependence, nicotine also causes psychological and social dependence which fundamentally depends on the personality configuration of each smoker. Nicotine produces a series of effects in the body among which increased blood pressure, increased heart rate or tachycardia, increase in glycemia and increased bowel movement, must be highlighted.

The most common genetic polymorphisms are SNPs and the copy-number variations (CNVs). As used herein, the term "polymorphism" refers to the substitutions of a single nucleotide with another and they are observed at a frequency greater than 1% in the general population. In a specific case, when said variation in the nucleotide sequence occurs in a single nucleotide (A, C, T or G), it is known as "SNP" (single-nucleotide polymorphism).

SNPs represent one of the most common forms of genetic variation, wherein each version of the sequence with respect to the polymorphic site is referred to as an allele of the polymorphic site. SNPs occur along the entire genome both in the extragenic regions and in the coding regions or the regulatory sequences thereof. The change in a single nucleotide can cause: 1) the substitution of an amino acid if it is in an exon sequence, and varying the efficacy of the protein function; 2) the modification of the amount of protein if the SNP is in a regulatory region; or 3) the change in protein structure if the change is in a splicing zone.

Mutations can be used as diagnostic and/or prognostic tools for identifying subjects with a predisposition to a disease or to a rapid evolution of the disease, genotyping the subject suffering the disease and facilitating the development of medicinal products based on the knowledge generated about the role of proteins in the pathogenesis process. Likewise, SNPs can also be used as diagnostic and/or prognostic tools for identifying subjects who respond well (or who do not respond) or who present adverse effects to a specific therapy or to a certain pharmacological treatment since the inherited differences between individuals in the drug pharmacokinetics and pharmacodynamics are due to genetic variants affecting the gene expression or function; these polymorphisms affect drug metabolizing enzymes, transporters and drug targets and can have a great influence on the efficacy and safety of a specific pharmacological treatment or therapy.

In some embodiments of the present invention, the genetic markers used in the invention are specific alleles in "polymorphic sites" associated with the safety of a nicotinic cholinergic receptor agonist drug-based pharmacological treatment. A nucleotide position in the genome in which more than one sequence is possible in a population is known as a "polymorphic site". When a polymorphic site is a single nucleotide long, the site is commonly called SNP as mentioned above, for example, if in a particular chromosomal location a member of a population has an adenine (A) and another member of the population has a thymine (T) in the same position, then this position is a polymorphic site and more specifically the polymorphic site is an SNP. Each version of the sequence with respect to the polymorphic site is known as an allele of the polymorphic site. Therefore, in the preceding example the SNP allows both an A allele and a T allele. These alleles are "allelic variants". Nucleotide sequence variants either in the coding or non-coding regions can give rise to changes in the coded polypeptide sequence, which affects the properties thereof (activity alteration, distribution alteration, altered stability, etc.). On the other hand, nucleotide sequence variants either in the coding or non-coding regions can give rise to changes affecting the transcription of a gene or the translation of its messenger RNA. In all cases, the alterations can be qualitative, quantitative, or both.

As used herein, the term "probability" measures the frequency with which a result (or set of results) is obtained upon carrying out a random experiment, of which all the possible results are known, under sufficiently stable conditions. As used herein, said term "probability" in relation to the safety of a nicotinic cholinergic receptor agonist-based pharmacological treatment refers to the propensity or the real probability of a subject to develop adverse effects in response to said pharmacological treatment which can lead to the withdrawal of said treatment or to a reduction of the usual dose of the drug used in said pharmacological treatment. The adverse effects can appear either at the start of the pharmacological treatment or later, for example, after 1, 2, 3, 4, 5, 6, 7, 8, 9, 10, 11, 12, 13, 14, 15 or more days (even weeks or months) after having started the pharmacological treatment. The probability that a subject will suffer adverse effects in response to a nicotinic cholinergic receptor agonist -based pharmacological treatment, which can lead to the withdrawal of said treatment or to a reduction of the usual dose of the drug used in said treatment, can be "high" or "low". As will be understood by the persons skilled in the art, the probability does not need to be 100% for all the evaluated subjects, although it would be preferably so. However, said term requires a statistically significant part of the subjects following a nicotinic cholinergic receptor agonist-based pharmacological treatment to be able to be identified as subjects having a high probability of obtaining a certain result, specifically, developing adverse effects. Whether or not a subject is statistically significant can be determined without major complications by a person skilled in the art using different known statistical evaluation tools, for example, by means of determining confidence intervals, determining p-value, Student's test, Mann-Whitney test, etc. Additional information about these statistical tools can be found in Dowdy and Wearden, Statistics for Research. John Wiley & Sons, New York 1983. The preferred confidence intervals are at least 50%, at least 60%, at least 70%, at least 80%, at least 90%, or at least 95%. The p-values are preferably 0.1, 0.05, 0.02, 0.01 or less.

As used herein, the term "subject" refers to a female or male human being of any race or age. In a particular embodiment, the subject is an active tobacco consumer, i.e., he/she consumes tobacco frequently or occasionally; in a more specific embodiment, said subject is an active smoker. Even though there is no unanimity regarding who should be considered as light, moderate or heavy smokers, nor regarding the exact limit separating the frequent smoker from the occasional smoker, by way of example, a smoker smoking more than 20 cigarettes a day could be considered as a "heavy" smoker, a smoker smoking between 10 and 20 cigarettes a day could be considered as a "moderate" smoker, and a smoker smoking less than 10 cigarettes a day could be considered as a "light" smoker. Likewise, even though the time necessary to consider a smoker as an "ex-smoker" has not been established, the period of one year of complete cessation or quitting tobacco consumption is most often considered. On the other hand, "non-smokers" are considered as those subjects who have not been actively or constantly exposed to tobacco.

As used herein, the term "tobacco" refers to a product processed from the leaves of several plants from the genus *Nicotiana,* among them *N. tabacum, N. petunoides, N rustica* and *N. polydiclia.* The species *N. tabacum* can be classified into four varieties, *havanensis, brasilensis, virginica* and *purpurea,* which are the origin of the different varieties used in marketing tobacco. The tobacco-based products, for which the methods of the invention relating to the prediction of successful or failed consumption cessation in subjects subjected to treatment with varenicline are intended, include those products made completely or partially of tobacco, which contain nicotine, and the consumption of which occur, for example, upon smoking them, sucking them, chewing them or sniffing them. In a non-limiting illustrative manner, the tobacco can be in the form of rolled tobacco [(also called cigarettes, cigs, etc.) which can be tipped or without filter, bright or dark, light, semi-light or whole], cigars, rolling tobacco, tobacco for pipe smoking, powdered tobacco or snuff, etc.

As used herein, the term "therapy" or "treatment" collectively refers to the means of any class, hygienic means, pharmacological means, surgical means or physical means, the purpose of which is to prevent and/or cure or relieve a disease or pathology or its symptoms. In a specific case, said treatment is a pharmacological treatment, i.e., a treatment comprising the administration of a drug to a subject to prevent, relieve and/or cure a disease or to relieve, reduce or eliminate one or more symptoms associated with said disease. In the context of the present invention, the treatment applied to the subject is a nicotinic cholinergic receptor agonist drug-based pharmacological type treatment and is intended for treating a pathology or disease which can be treated with said drug. In a particular embodiment, said drug is varenicline and the pathology to be treated is nicotine or tobacco addiction or tobaccoism. In a particular embodiment, the subject for whom the treatment is intended is an active tobacco consumer, for example, an active smoker.

As used herein, the term "customized treatment" or "customized medicine" refers to the design and application of interventions for prevention, diagnosis and treatment adapted to the genetic substrate of the patient and to the molecular profile of the disease.

As used herein, the term "varenicline" refers to the compound 7,8,9,10-tetrahydro-6,10-methane-6H-pyrazino[2,3-h][3]benzazepine of formula: 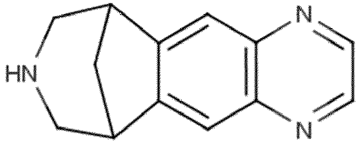 and also includes its pharmaceutically acceptable salts, such as those mentioned in international patent application WO99/35131, for example, as well as the derivatives thereof, for example, N-formyl-varenicline and N-methyl-varenicline. Varenicline is marketed in Spain in the form of tartrate salt under the brand Chantix^{®} or Champix^{®}.

Varenicline is comprised within the group of nicotinic cholinergic receptor agonist drugs and is a compound which acts as a selective partial agonist of nicotinic acetylcholine receptors which binds with high affinity and selectivity to the α4β2 neuronal nicotinic acetylcholine receptor subtype.

Varenicline is used in the treatment of various diseases including, in a non-limiting illustrative manner, chronic inflammatory bowel diseases (including, without limitation, ulcerative colitis, pyoderma gangrenosum and Crohn's disease), irritable bowel syndrome, spasmodic dystonia, chronic pain, acute pain, celiac disease, pouchitis, vasoconstriction, anxiety, panic disorder, depression, bipolar disorder, autism, sleep disorder, jet lag, amyotrophic lateral sclerosis (ALS), cognitive dysfunction, hypertension, bulimia, anorexia, obesity, heart arrhythmia, gastric acid hypersecretion, ulcer, pheochromocytoma, progressive supranuclear palsy, chemical addiction and dependence (nicotine, tobacco products, alcohol, benzodiazepine, barbiturates, opioids, cocaine), headache, migraine, stroke, traumatic brain injury (TBI), obsessive-compulsive disorder, psychosis, Huntington's disease, tardive dyskinesia, hyperkinesia, dyslexia, schizophrenia, multi-infarct dementia, age-associated cognitive impairment, epilepsy, Alzheimer's disease senile dementia, Parkinson's disease, attention deficit-hyperactivity disorder (ADHD) and Tourette syndrome.

The usual treatment for tobacco consumption cessation with varenicline comprises orally administering 1 mg of the compound twice a day for 12 weeks according to the following protocol:
- week 1: 0.5 milligrams (mg) once a day for days 1 to 3 of treatment and 0.5 mg twice a day for days 4 to 7 of treatment;
- week 2: 1 mg twice a day for days 8 to 14 of treatment;
- week 3 to 12: 1 mg twice a day starting from day 15 of treatment and to the end of the treatment.

The absorption is complete and the systemic availability is high. The maximum plasma concentrations typically occur 3-4 hours after intake (Faessel HM et al. 2010 Clin Pharmacokinet 49(12) : 799-816) .

### Method for determining the probability that a subject will suffer adverse effects in response to a nicotinic cholinergic receptor agonist drug-based therapy ("first method of the invention")

In one aspect, the invention relates to a method for determining the probability that a subject will suffer adverse effects in response to a nicotinic cholinergic receptor agonist drug-based therapy (hereinafter, "first method of the invention"), wherein said adverse effects lead to the withdrawal of said therapy or to a reduction of the usual therapeutic dose of the drug used in said therapy, said method comprising determining in a biological sample of said subject at least one allele of one or more of single-nucleotide polymorphisms (SNPs) rs7930792, rs12423809, rs4251417, rs7146, rs477292, rs495491, rs3778151, rs9479757, rs763132, rs4474069 and rs1183035, wherein
- the presence of at least one C allele of SNP rs7930792,
- the presence of at least one C allele of SNP rs12423809,
- the presence of at least one A allele of SNP rs4251417,
- the presence of at least one T allele of SNP rs7146,
- the presence of at least one A allele of SNP rs477292,
- the presence of at least one C allele of SNP rs495491,
- the presence of at least one C allele of SNP rs3778151,
- the presence of at least one A allele of SNP rs9479757,
- the presence of at least one T allele of SNP rs763132,
- the presence of at least one C allele of SNP rs4474069, and/or
- the presence of at least one T allele of SNP rs1183035,
is indicative that said subject has a high probability of suffering adverse effects in response to said nicotinic cholinergic receptor agonist drug-based therapy that lead to the withdrawal of said therapy or to a reduction of the usual therapeutic dose of the drug used in said therapy.

In an alternative embodiment of the first method of the invention, the invention also relates to a method for determining the probability that a subject will suffer adverse effects in response to a nicotinic cholinergic receptor agonist drug-based therapy, which comprises determining in a biological sample of said subject at least one allele of one or more of SNPs rs7930792, rs12423809, rs4251417, rs7146, rs477292, rs495491, rs3778151, rs9479757, rs763132, rs4474069 or rs1183035, wherein
- the presence of at least one G allele of SNP rs7930792,
- the presence of at least one A allele of SNP rs12423809,
- the presence of at least one G allele of SNP rs4251417,
- the presence of at least one C allele of SNP rs7146,
- the presence of at least one G allele of SNP rs477292,
- the presence of at least one T allele of SNP rs495491,
- the presence of at least one T allele of SNP rs3778151,
- the presence of at least one G allele of SNP rs9479757,
- the presence of at least one G allele of SNP rs763132,
- the presence of at least one T allele of SNP rs4474069, and/or
- the presence of at least one C allele of SNP rs1183035,
is indicative that said subject has a low probability of suffering adverse effects in response to said nicotinic cholinergic receptor agonist drug-based therapy that lead to the withdrawal of said therapy or to a reduction of the usual therapeutic dose of the drug used in said therapy.

Non-limiting illustrative examples of adverse effects of nicotinic cholinergic receptor agonist drugs include the adverse effects mentioned above in the definition of "varenicline" within the section "Definitions". Among said adverse effects, the adverse effects that lead to the withdrawal of the nicotinic cholinergic receptor agonist drug-based therapy or to a reduction of the usual therapeutic dose of the drug used in said therapy, are found.

In a particular embodiment, said adverse effects in response to a nicotinic cholinergic receptor agonist drug-based therapy, such as varenicline for example, that lead to the withdrawal of said therapy or to a reduction of the usual therapeutic dose of the drug used in said therapy are selected from the group consisting of insomnia, nocturnal agitation, drowsiness, vivid dreams, headaches, dizziness, restlessness, nervous tension, symptoms of depression, bloated feeling in the stomach, abdominal distension, meteorism, flatulence, nausea, vomiting, diarrhea, constipation, dry mouth, aphthae, skin rash, fatigue, general aches and pains and combinations thereof.

According to the first method of the invention, in a biological sample of the subject under study at least one allele of one or more of SNPs rs7930792, rs12423809, rs4251417, rs7146, rs477292, rs495491, rs3778151, rs9479757, rs763132, rs4474069 and rs1183035, is determined, and the results obtained are correlated with the (high or low) probability that the subject under study will suffer adverse effects in response to a nicotinic cholinergic receptor agonist drug-based therapy, wherein said adverse effects lead to the withdrawal of said therapy or to a reduction of the usual therapeutic dose of the drug used in said therapy. Non-limiting illustrative examples of nicotinic cholinergic receptor agonist drugs include cytisine, dianicline and varenicline.

In a particular embodiment, said nicotinic cholinergic receptor agonist drug is varenicline. The varenicline-based therapy is administered to a subject suffering a disease or pathology which can be treated by means of varenicline; non-limiting illustrative examples of said diseases include the diseases and pathologies mentioned above in the definition of "varenicline" within the section "Definitions". In a particular embodiment, said varenicline-based therapy is administered to a subject suffering a chemical addiction or dependence, particularly, a nicotine or tobacco addiction (or dependence). The varenicline-based therapy administered to a subject can help him/her to stop consuming tobacco, or to relieve the anxiety and abstinence syndrome associated with stopping tobacco consumption, or to reduce the pleasure caused by tobacco.

The subject is a human being of any sex, race or age; nevertheless, in a particular embodiment, the subject is an active tobacco consumer, for example, a cigarette, electronic cigarette, cigar or pipe smoker, or a powdered tobacco or snuff consumer. In yet another more particular embodiment, said subject is an active tobacco consumer who wants to stop consuming tobacco voluntarily, or who must stop consuming tobacco due to medical prescription, for example. In another particular embodiment, the subject is a subject suffering a disease which can be treated with a nicotinic cholinergic receptor agonist drug different from nicotine or tobacco addiction (or dependence).

The biological sample coming from the subject under study is a biological sample containing a nucleic acid, for example, DNA, genomic DNA (gDNA), complementary DNA (cDNA), RNA, heterogeneous nuclear RNA (hnRNA), mRNA, etc., of the subject to be evaluated. Said biological sample can be isolated or extracted from a tissue or can be present in a biological fluid, for example, blood, saliva, etc. The methods for isolating biological samples from cells and tissues are well known by the persons skilled in the art. In a particular embodiment, said biological sample is a blood or saliva sample.

Generally, when the biological sample is a complete blood sample, it can directly be used in putting the first method of the invention into practice. However, on other occasions, it is necessary to first extract the nucleic acid from the biological samples, for example, cells present in a biological fluid; in that case, all the nucleic acids extracted from said biological samples represent the working material. The isolation of nucleic acids from a biological sample containing same can be performed by methods known by the persons skilled in the art [see, for example, Sambrook et al, 2001 "Molecular Cloning: A Laboratory Manual", 3rd edition, Cold Spring Harbor Laboratory Press, New York, vol. 1.3]. Likewise, it may sometimes be necessary to amplify the extracted nucleic acid before analysis. Various nucleic acid amplification methods are known; many of them are based on an enzymatic reaction, for example, the polymerase chain reaction (PCR), the ligase chain reaction (LCR), rolling-circle amplification assays, etc. Information about said nucleic acids amplification methods can be found in Sambrook *et al,* 2001 (mentioned *supra*)*.*

After isolating and amplifying (if necessary) the nucleic acid, one or more of SNPs rs7930792, rs12423809, rs4251417, rs7146, rs477292, rs495491, rs3778151, rs9479757, rs763132, rs4474069 and rs1183035, is analyzed, and the nucleotides present in said SNPs, particularly the nucleotides present in the polymorphic site of the SNP which allows identifying the corresponding allele of the SNP, are identified.

The persons skilled in the art will readily recognize that the analysis of the nucleotides present in one or more of said SNPs in the sample containing nucleic acid of the subject under study can be performed by any method or technique capable of determining the nucleotides present in a polymorphic site (e.g., SNP), for example, by means of sequencing, minisequencing, hybridization, restriction fragment analysis, oligonucleotide ligation assays, allele-specific PCR, HRM (High Resolution Melt) analysis, etc., or by means of any combination of said methods. The person skilled in the art can use any suitable method or technique to achieve that determination. The nucleotides present in the SNPs and particularly in the polymorphic sites can be detected from any nucleic acid strand or from both strands.

Non-limiting illustrative examples of methods, techniques or systems suitable for analyzing the nucleotides present in the polymorphic sites of the SNPs identified in the present invention include the methods of minisequencing, nucleic acid sequencing, hybridization, restriction fragment analysis, oligonucleotide ligation assays, allele-specific PCR, HRM analysis, DNA arrays, for example, the technology available in Aclara BioSciences, Affymetrix, Agilent Technologies, Illumina Inc., Ion Torrent, Fluidigm, NanoporeTech etc., techniques based on the change in the mobility of amplified nucleic acid fragments, single-strand conformation polymorphisms (SSCPs), denaturing gradient gel electrophoresis (DGGE), CMC (chemical mismatch cleavage), WAVE analysis, etc., or by means of any combination of said methods. Additional information about the analysis of the nucleotides present in the polymorphic sites of the SNPs can be found in Sambrook *et al.,* 2001, mentioned *supra,* as well as in United States patent application US2007/0105128, for example.

According to the first method of the invention, at least one allele of one or more of SNPs rs7930792, rs12423809, rs4251417, rs7146, rs477292, rs495491, rs3778151, rs9479757, rs763132, rs4474069 and rs1183035, in the biological sample of the subject under study, is genotyped or determined.

Therefore, in a particular embodiment, the first method of the invention comprises determining a single allele of one or more of SNPs rs7930792, rs12423809, rs4251417, rs7146, rs477292, rs495491, rs3778151, rs9479757, rs763132, rs4474069 and rs1183035, i.e., determining the nucleotide present in said SNP. Therefore, in a particular embodiment, a single allele of SNP rs7930792 is determined; in another particular embodiment, a single allele of SNP rs12423809 is determined; in another particular embodiment, a single allele of SNP rs4251417 is determined; in another particular embodiment, a single allele of SNP rs7146 is determined; in another particular embodiment, a single allele of SNP rs477292 is determined; in another particular embodiment, a single allele of SNP rs495491 is determined; in another particular embodiment, a single allele of SNP rs3778151 is determined; in another particular embodiment, a single allele of SNP rs9479757 is determined; in another particular embodiment, a single allele of SNP rs763132 is determined; in another particular embodiment, a single allele of SNP rs4474069 is determined; in another particular embodiment, a single allele of SNP rs1183035 is determined; whereas, in another particular embodiment, a single allele of any of the SNPs of the corresponding linkage groups of SNPs rs7930792, rs12423809, rs4251417, rs7146, rs477292, rs495491, rs3778151, rs9479757, rs763132, rs4474069 or rs1183035 is determined. Information about the linkage groups of said SNPs will be provided below. The person skilled in the art can obtain information about the SNPs present in a linkage group of a certain SNP using suitable databases, for example, the International HapMap Project database (www.hapmap.org and http://hapmap.ncbi.nlm.nih.gov/) and/or suitable programs such as the Haploview program using the confidence interval or Gabriel algorithm method (Gabriel et al., Science, 2002, 296(5576):225-9) or any other more up-to-date version (Barrett et al., 2005, Bioinformatics 21(2):263-265).

In another particular embodiment, the first method of the invention comprises determining the two alleles of one or more of said SNPs rs7930792, rs12423809, rs4251417, rs7146, rs477292, rs495491, rs3778151, rs9479757, rs763132, rs4474069 and rs1183035. Therefore, in a particular embodiment, the two alleles of SNP rs7930792 are determined; in another particular embodiment, the two alleles of SNP rs12423809 are determined; in another particular embodiment, the two alleles of SNP rs4251417 are determined; in another particular embodiment, the two alleles of SNP rs7146 are determined; in another particular embodiment, the two alleles of SNP rs477292 are determined; in another particular embodiment, the two alleles of SNP rs495491 are determined; in another particular embodiment, the two alleles of SNP rs3778151 are determined; in another particular embodiment, the two alleles of SNP rs9479757 are determined; in another particular embodiment, the two alleles of SNP rs763132 are determined; in another particular embodiment, the two alleles of SNP rs4474069 are determined; in another particular embodiment, the two alleles of SNP rs1183035 are determined. In another particular embodiment, the first method of the invention comprises determining one or both alleles of any 1, 2, 3, 4, 5, 6, 7, 8, 9, 10 or 11 SNPs of SNPs rs7930792, rs12423809, rs4251417, rs7146, rs477292, rs495491, rs3778151, rs9479757, rs763132, rs4474069 and rs1183035, whereas in another particular embodiment, said method comprises determining one or both alleles of the 11 SNPs rs7930792, rs12423809, rs4251417, rs7146, rs477292, rs495491, rs3778151, rs9479757, rs763132, rs4474069 and rs1183035. The SNPs identified in the present invention associated with the (high or low) probability that a subject will suffer adverse effects in response to a nicotinic cholinergic receptor agonist drug-based therapy, wherein said adverse effects lead to the withdrawal of said therapy or to a reduction of the usual therapeutic dose of the drug used in said therapy, or as will be mentioned below, with the prediction of the safety of a nicotinic cholinergic receptor agonist drug-based therapy to quit tobacco consumption, or with the selection of a subject to follow a nicotinic cholinergic receptor agonist drug-based therapy, have the following characteristics:
- SNP rs7930792 is located in the *UBASH3B* (*ubiquitin associated and SH3 domain containing B)* gene in the human chromosome 11 (11q24.1), corresponds to the sequence TCTTGTTTCAGAAAATAAGGTCCCAG[C/G]AAAGTCAATGGGTTAGGAAGTGT GG (SEQ ID NO: 1), and is located in the linkage group extending from nucleotide 122580938 to nucleotide 122588149;
- SNP rs12423809 is located in the *C12orf36* (*chromosome 12 open reading frame 36)* gene in the human chromosome 12, corresponds to the sequence AGGAGAAAGATGAATATCCCAGCTCA[A/C]GCAGCCAACAAATTTGCCCTTCC TC (SEQ ID NO: 2), and is located in the linkage group extending from nucleotide 13528225 to nucleotide 13535853;
- SNP rs4251417 is located in the *SLC6A4* (*solute carrier family 6 member 4)* gene in the human chromosome 17 (17q11.1-q12), corresponds to the sequence: ACCCCTGAGGACTCCTGAGAACACAC[A/G]TTGCCCTCAAAAATCTACCTACC TG (SEQ ID NO: 3), and is located in the linkage group extending from nucleotide 28511978 to nucleotide 28552773;
- SNP rs7146 is located in the *C19orf6* (*chromosome 19 open reading frame 6)* gene in the human chromosome 19, corresponds to the sequence: CGCTCGCCCATCAACTGCCTGGAGCA[C/T]GTGCGTGACAAGTGGCCGCGTGA GG (SEQ ID NO: 4), and is located in the linkage group extending from nucleotide 1008879 to nucleotide 1020083;
- SNP rs477292 is located in the *OPRM1 (opioid receptor mu 1*) gene in the human chromosome 6 (6q24-q25), corresponds to the sequence: TCAGCTAAATAGACATACTTGCTTCA[A/G]TCCCCAGATGCTTTCCCTGTCGC TT (SEQ ID NO: 5), and is located in the linkage group extending from nucleotide 154362019 to nucleotide 154406051;
- SNP rs495491 is located in the *OPRM1 (opioid receptor mu 1*) gene in the human chromosome 6 (6q24-q25), corresponds to the sequence: TGATAGGCACTGGTTCTACAGTGAGA[C/T]ATATCTCTCCTAAGTCTGGTGAC AA (SEQ ID NO: 6), and is located in the linkage group extending from nucleotide 154362019 to nucleotide 154406051;
- SNP rs3778151 is located in the *OPRM1 (opioid receptor mu 1*) gene in the human chromosome 6 (6q24-q25), corresponds to the sequence: TCAAGATAGCTAATTGAGAACAAGCA[C/T]GAGACTCCACTCCTGGTCCCCAA GC (SEQ ID NO: 7), and is located in the linkage group extending from nucleotide 154362019 to nucleotide 154406051;
- the SNP rs9479757 is located in the *OPRM1* gene (*opioid receptor mu 1*), in the human chromosome 6 (6q24-q25), corresponds to the sequence: GTGATGTTACCAGCCTGAGGGAAGGA[A/G]GGTTCACAGCCTGATATGTTGGT GA (SEQ ID NO: 8), and is located in the linkage group extending from nucleotide 154410114 to nucleotide 154451287;
- SNP rs763132 is located in the *ARHGAP18* (*Rho GTPase activating protein 18*) gene in the human chromosome 6 (6q22-q24), corresponds to the sequence: AAGGTGGAAAATGACATACATTCTTG[G/T]GACTTTAGATGAATCAGATTATA GC (SEQ ID NO: 9).
- SNP rs4474069 is located in the *RAPGEF1* (*Rap guanine nucleotide exchange factor 1*) gene in the human chromosome 9 (9q34.3), corresponds to the sequence: TCTCACTGTCTGTAATGACTCCAGCA[C/T]GAAAAGCTCCCAGCCACAGGCAG CT (SEQ ID NO: 10), and is located in the linkage group extending from nucleotide 134486977 to nucleotide 134590774; and
- SNP rs1183035 is located in the *MAOB* (*monoamine oxidase B*) gene in the human chromosome X (Xp11.23), corresponds to the sequence: TCACTGTAGAACTAGAAAAATGGTCA[C/T]TTATTGCAAAGTGCTACAGTGAA AA (SEQ ID NO: 11), and is located in the linkage group extending from nucleotide 43689236 to nucleotide 43754959.

The SNPs rs7930792, rs12423809, rs4251417, rs7146, rs477292, rs495491, rs3778151, rs9479757, rs763132, rs4474069 and rs1183035 according to the present invention correspond to the SNPs listed in the NCBI database of single-nucleotide polymorphisms (dbSNP, NCBI, http://www.ncbi.nlm.nih.gov/snp; March 2011) and according to HapMap Genome Browser (version 24, Phase 1&2) The indicated nucleotide positions for the linkage groups are defined according to the Haploview software version 4.2 (corresponding to the version of September 2009). The polymorphic sites (nucleotide positions within the genome in which there can be more than one nucleotide sequence in a population) present in said SNPs rs7930792, rs12423809, rs4251417, rs7146, rs477292, rs495491, rs3778151, rs9479757, rs763132, rs4474069 and rs1183035 referred to in the present invention are the following:
- the polymorphic site of SNP rs7930792 corresponds to position 27 of the sequence SEQ ID NO: 1;
- the polymorphic site of SNP rs12423809 corresponds to position 27 of the sequence SEQ ID NO: 2;
- the polymorphic site of SNP rs4251417 corresponds to position 27 of the sequence SEQ ID NO: 3;
- the polymorphic site of SNP rs7146 corresponds to position 27 of the sequence SEQ ID NO: 4;
- the polymorphic site of SNP rs477292 corresponds to position 27 of the sequence SEQ ID NO: 5;
- the polymorphic site of SNP rs495491 corresponds to position 27 of the sequence SEQ ID NO: 6;
- the polymorphic site of SNP rs3778151 corresponds to position 27 of the sequence SEQ ID NO: 7;
- the polymorphic site of SNP rs9479757 corresponds to position 27 of the sequence SEQ ID NO: 8;
- the polymorphic site of SNP rs763132 corresponds to position 27 of the sequence SEQ ID NO: 9;
- the polymorphic site of SNP rs4474069 corresponds to position 27 of the sequence SEQ ID NO: 10; and
- the polymorphic site of SNP rs1183035 corresponds to position 27 of the sequence SEQ ID NO: 11.

The specific nucleotide present in the polymorphic site of said SNPs rs7930792, rs12423809, rs4251417, rs7146, rs477292, rs495491, rs3778151, rs9479757, rs763132, rs4474069 and rs1183035 allows identifying the corresponding allele; therefore, the C allele of SNP rs7930792 corresponds to the allele in which there is a C in the polymorphic site of said SNP; the C allele of SNP rs12423809 corresponds to the allele in which there is a C in the polymorphic site of said SNP; the A allele of SNP rs4251417 corresponds to the allele in which there is an A in the polymorphic site of said SNP; the T allele of SNP rs7146 corresponds to the allele in which there is a T in the polymorphic site of said SNP; the A allele of SNP rs477292 corresponds to the allele in which there is an A in the polymorphic site of said SNP; the C allele of SNP rs495491 corresponds to the allele in which there is a C in the polymorphic site of said SNP; the C allele of SNP rs3778151 corresponds to the allele in which there is a C in the polymorphic site of said SNP; the A allele of SNP rs9479757 corresponds to the allele in which there is an A in the polymorphic site of said SNP; the T allele of SNP rs763132 corresponds to the allele in which there is a T in the polymorphic site of said SNP; the C allele of SNP rs4474069 corresponds to the allele in which there is a C in the polymorphic site of said SNP; the T allele of SNP rs1183035 corresponds to the allele in which there is a T in the polymorphic site of said SNP.

Studies conducted by the inventors (Example 1) have allowed identifying the nucleotides present in the polymorphic sites of the SNPs described in this document associated with a high probability that the subject will suffer adverse effects in response to a varenicline-based therapy that lead to the withdrawal of said therapy or to a reduction of the usual therapeutic dose of the drug used in said therapy, sometimes referred to in this description as "alleles associated with a high probability of the onset of adverse effects in the subject in response to a nicotinic cholinergic receptor agonist drug-based therapy" or simply as "alleles associated with adverse effects". Said "alleles associated with adverse effects" are listed in Table 1.

**Table 1**

| Alleles associated with a high probability that a subject will suffer adverse effects in response to a nicotinic cholinergic receptor agonist drug-based therapy ("alleles associated with adverse effects") for the different SNPs identified in the present invention | |
|---|---|
| SNP | Allele associated with adverse effects |
| rs7930792 | C |
| rs12423809 | C |
| rs4251417 | A |
| rs7146 | T |
| rs477292 | A |
| rs495491 | C |
| rs3778151 | C |
| rs9479757 | A |
| rs763132 | T |
| rs4474069 | C |
| rs1183035 | T |

The alleles associated with adverse effects indicated in Table 1 correspond to the TOP strand of the DNA according to the Illumina nomenclature for DNA strand identification. As is known, the simplest case for designating a strand occurs when one of the possible variations of the SNP is an adenine (A), and the remaining variation is either a cytosine (C) or a guanine (G). In that case, the sequence of that SNP is designated TOP. Similar to the rules of reverse complementarity, when one of the possible variations of the SNP is a thymine (T), and the remaining variation is either a C or a G, the sequence of this SNP is designated BOT. If the SNP is an [A/T] or a [C/G], then the preceding rules do not apply. Illumina uses a "sequence walking" technique for designating the strand [A/T] and [C/G] of the SNPs. For this sequence walking method, the actual SNP is considered to be in position 'n' . The sequences immediately before and after the SNP are 'n-1' and 'n+1', respectively. Similarly, two base pairs before the SNP is 'n-2' and two base pairs after the SNP is 'n+2', etc. Sequence walking continues using this method until an unambiguous pairing (A/G, A/C, T/C, or T/G) is present. To designate the strand, when the nucleotide A or T of the first unambiguous pair is on the 5' side of the SNP, then the sequence is designated TOP. When the nucleotide A or T of the first unambiguous pair is on the 3' side of the SNP, then the sequence is designated BOT.

According to the first method of the invention, once at least one of the alleles of one or more of SNPs rs7930792, rs12423809, rs4251417, rs7146, rs477292, rs495491, rs3778151, rs9479757, rs763132, rs4474069 and rs1183035, is determined in a biological sample of said subject, it is possible to determine the probability that the subject under study will suffer adverse effects in response to a nicotinic cholinergic receptor agonist drug-based therapy, for example, varenicline, cytisine, dianicline, or any other drug with a similar mechanism of action, that lead to the withdrawal of said therapy or to a reduction of the usual therapeutic dose of the drug used in said therapy. The establishment of the (high or low) probability that the subject under study will suffer such adverse effects in response to a nicotinic cholinergic receptor agonist drug-based therapy can be carried out based on the presence or absence of the alleles associated with adverse effects (i.e., based on the presence or absence of the nucleotides present in the polymorphic sites of said SNPs associated with a high or low probability that the subject under study will suffer said adverse effects in response to a nicotinic cholinergic receptor agonist drug-based therapy) of said SNPs in the genome of the subject under study. Therefore, as mentioned above:
- the presence of at least one C allele of SNP rs7930792,
- the presence of at least one C allele of SNP rs12423809,
- the presence of at least one A allele of SNP rs4251417,
- the presence of at least one T allele of SNP rs7146,
- the presence of at least one A allele of SNP rs477292,
- the presence of at least one C allele of SNP rs495491,
- the presence of at least one C allele of SNP rs3778151,
- the presence of at least one A allele of SNP rs9479757,
- the presence of at least one T allele of SNP rs763132,
- the presence of at least one C allele of SNP rs4474069, and/or
- the presence of at least one T allele of SNP rs1183035, is indicative that said subject has a high probability of suffering adverse effects in response to said nicotinic cholinergic receptor agonist drug-based therapy that lead to the withdrawal of said therapy or to a reduction of the usual therapeutic dose of the drug used in said therapy.

Likewise, in a particular embodiment of the first method of the invention:
- the presence of both C alleles of SNP rs7930792,
- the presence of both C alleles of SNP rs12423809,
- the presence of both A alleles of SNP rs4251417,
- the presence of both T alleles of SNP rs7146,
- the presence of both A alleles of SNP rs477292,
- the presence of both C alleles of SNP rs495491,
- the presence of both C alleles of SNP rs3778151,
- the presence of both A alleles of SNP rs9479757,
- the presence of both T alleles of SNP rs763132,
- the presence of both C alleles of SNP rs4474069, and/or
- the presence of both T alleles of SNP rs1183035,
is indicative that said subject has a high probability of suffering adverse effects in response to said nicotinic cholinergic receptor agonist drug-based therapy that lead to the withdrawal of said therapy or to a reduction of the usual therapeutic dose of the drug used in said therapy.

As used herein, the term "high" applied to the probability that a subject will suffer adverse effects in response to a nicotinic cholinergic receptor agonist drug-based therapy, wherein said adverse effects lead to the withdrawal of said therapy or to a reduction of the usual therapeutic dose of the drug used in said therapy refers to the fact that the frequency with which a result is obtained, in this case, the frequency with which a subject will suffer such adverse effects in response to a treatment with a nicotinic cholinergic receptor agonist drug, is greater than 50%.

Alternatively, as mentioned above, based on the results obtained in the present invention, it can be established that:
- the presence of at least one G allele of SNP rs7930792,
- the presence of at least one A allele of SNP rs12423809,
- the presence of at least one G allele of SNP rs4251417,
- the presence of at least one C allele of SNP rs7146,
- the presence of at least one G allele of SNP rs477292,
- the presence of at least one T allele of SNP rs495491,
- the presence of at least one T allele of SNP rs3778151,
- the presence of at least one G allele of SNP rs9479757,
- the presence of at least one G allele of SNP rs763132,
- the presence of at least one T allele of SNP rs4474069,
- the presence of at least one C allele of SNP rs1183035,
is indicative that said subject has a low probability of suffering adverse effects in response to said nicotinic cholinergic receptor agonist drug-based therapy that lead to the withdrawal of said therapy or to a reduction of the usual therapeutic dose of the drug used in said therapy.

The specific nucleotide present in the polymorphic site of said SNPs rs7930792, rs12423809, rs4251417, rs7146, rs477292, rs495491, rs3778151, rs9479757, rs763132, rs4474069 and rs1183035 allows identifying the corresponding allele; therefore, the G allele of SNP rs7930792 corresponds to the allele in which there is a G in the polymorphic site of said SNP; the A allele of SNP rs12423809 corresponds to the allele in which there is an A in the polymorphic site of said SNP; the G allele of SNP rs4251417 corresponds to the allele in which there is a G in the polymorphic site of said SNP; the C allele of SNP rs7146 corresponds to the allele in which there is a C in the polymorphic site of said SNP; the G allele of SNP rs477292 corresponds to the allele in which there is a G in the polymorphic site of said SNP; the T allele of SNP rs495491 corresponds to the allele in which there is a T in the polymorphic site of said SNP; the T allele of SNP rs3778151 corresponds to the allele in which there is a T in the polymorphic site of said SNP; the G allele of SNP rs9479757 corresponds to the allele in which there is a G in the polymorphic site of said SNP; the G allele of SNP rs763132 corresponds to the allele in which there is a G in the polymorphic site of said SNP; the T allele of SNP rs4474069 corresponds to the allele in which there is a T in the polymorphic site of said SNP; the C allele of SNP rs1183035 corresponds to the allele in which there is a C in the polymorphic site of said SNP.

More specifically, in a more particular embodiment of this alternative:
- the presence of both G alleles of SNP rs7930792,
- the presence of both A alleles of SNP rs12423809,
- the presence of both G alleles of SNP rs4251417,
- the presence of both C alleles of SNP rs7146,
- the presence of both G alleles of SNP rs477292,
- the presence of both T alleles of SNP rs495491,
- the presence of both T alleles of SNP rs3778151,
- the presence of both G alleles of SNP rs9479757,
- the presence of both G alleles of SNP rs763132,
- the presence of both T alleles of SNP rs4474069,
- the presence of both C alleles of SNP rs1183035,
is indicative that said subject has a high probability of suffering adverse effects in response to said nicotinic cholinergic receptor agonist drug-based therapy that lead to the withdrawal of said therapy or to a reduction of the usual therapeutic dose of the drug used in said therapy.

As used herein, the term "low" applied to the probability that a subject will suffer adverse effects in response to a nicotinic cholinergic receptor agonist drug-based therapy, wherein said adverse effects lead to the withdrawal of said therapy or to a reduction of the usual therapeutic dose of the drug used in said therapy refers to the fact that the frequency with which a result is obtained, in this case, the frequency with which a subject will suffer adverse effects in response to a treatment with a nicotinic cholinergic receptor agonist drug, is less than 50%.

Additionally, assays performed by the inventors have clearly shown that the SNPs analyzed according to the first method of the invention can be classified into two groups:
- Group 1: formed by SNPs rs7930792, rs12423809, rs4251417, rs7146, rs477292, rs495491, rs3778151, rs9479757; these SNPs are linked to a high probability that a subject will suffer adverse effects in response to a nicotinic cholinergic receptor agonist drug-based therapy that lead to the withdrawal or suspension of said therapy due to said adverse effects; and
- Group 2: formed by SNPs rs763132, rs4474069 and rs1183035; these SNPs are linked to a high probability that a subject will suffer adverse effects in response to a nicotinic cholinergic receptor agonist drug-based therapy that lead to reduction of the dose of the drug used in said therapy due to said adverse effects.

In a more specific embodiment, the first method of the invention contemplates the possibility of analyzing in a biological sample of said subject the presence or absence of at least one of the alleles of one or more of SNPs rs7930792, rs12423809, rs4251417, rs7146, rs477292, rs495491, rs3778151, rs9479757, wherein
- the presence of at least one C allele of SNP rs7930792,
- the presence of at least one C allele of SNP rs12423809,
- the presence of at least one A allele of SNP rs4251417,
- the presence of at least one T allele of SNP rs7146,
- the presence of at least one A allele of SNP rs477292,
- the presence of at least one C allele of SNP rs495491,
- the presence of at least one C allele of SNP rs3778151, and/or
- the presence of at least one A allele of SNP rs9479757,
is indicative that said subject has a high probability of suffering adverse effects in response to said nicotinic cholinergic receptor agonist drug-based therapy that lead to the withdrawal or suspension of said therapy.

In another specific embodiment, the first method of the invention contemplates the possibility of analyzing in a biological sample of said subject the presence or absence of at least one of the alleles of one or more of SNPs rs7930792, rs12423809, rs4251417, rs7146, rs477292, rs495491, rs3778151, rs9479757, wherein
- the presence of both C alleles of SNP rs7930792,
- the presence of both C alleles of SNP rs12423809,
- the presence of both A alleles of SNP rs4251417,
- the presence of both T alleles of the SNP rs7146,
- the presence of both A alleles of SNP rs477292,
- the presence of both C alleles of SNP rs495491,
- the presence of both C alleles of SNP rs3778151, and/or
- the presence of both A alleles of SNP rs9479757,
is indicative that said subject has a high probability of suffering adverse effects in response to said nicotinic cholinergic receptor agonist drug-based therapy that lead to the withdrawal or suspension of said therapy.

When the probability that a subject will suffer adverse effects in response to a nicotinic cholinergic receptor agonist drug-based therapy that lead to the withdrawal or suspension of said therapy is high, administering a therapy based on said drug causing the onset of said adverse effects to the subject and the administration of which would lead to the withdrawal or suspension of said therapy would be avoided; in such a situation, the specialist would select another alternative therapy. The possibility of withdrawing from said nicotinic cholinergic receptor agonist drug-based therapy would thus be substantially reduced, whereby the subject would maintain a suitable therapeutic adherence, and furthermore, the health expenditure would be optimized.

Likewise, in another more specific embodiment, the first method of the invention contemplates the possibility of analyzing in a biological sample of said subject the presence or absence of at least one allele of one or more of SNPs rs763132, rs4474069 or rs1183035, or any combination of said SNPs, wherein
- the presence of at least one T allele of SNP rs763132,
- the presence of at least one C allele of SNP rs4474069, and/or
- the presence of at least one T allele of SNP rs1183035,
is indicative that said subject has a high probability of suffering adverse effects in response to said nicotinic cholinergic receptor agonist drug-based therapy that lead to a reduction of the usual therapeutic dose of the drug used in said therapy.

Likewise, in another more specific embodiment, the first method of the invention contemplates the possibility of analyzing in a biological sample of said subject the presence or absence of at least one allele of one or more of SNPs rs763132, rs4474069 or rs1183035, wherein
- the presence of both T alleles of SNP rs763132,
- the presence of both C alleles of SNP rs4474069, and/or
- the presence of both T alleles of SNP rs1183035,
is indicative that said subject has a high probability of suffering adverse effects in response to said nicotinic cholinergic receptor agonist drug-based therapy that lead to a reduction of the usual therapeutic dose of the drug used in said therapy.

When the probability that a subject will suffer adverse effects in response to a nicotinic cholinergic receptor agonist drug-based therapy that lead to a reduction of the usual therapeutic dose of the drug used in said therapy is high, administering the usual therapeutic dose of said drug causing the onset of said adverse effects to the subject and the administration of which would lead to the reduction of the usual therapeutic dose of said drug would be avoided. The new dose to be administered in that case, which could be half, one third, one quarter, one fifth, one sixth, one seventh, one eighth, one ninth, or one tenth of the usual therapeutic dose, or even less, would depend on various factors, for example, the disease to be treated, seriousness of the adverse effects, general state of health of the subject under treatment, etc., and the adjustment thereof would be left to the discretion of the specialist who could alternatively choose another pharmacological therapy. The possibility of withdrawing from said nicotinic cholinergic receptor agonist drug-based therapy would thus be substantially reduced, whereby the subject would maintain a suitable therapeutic adherence, and furthermore, the health expenditure would be optimized.

The first method of the invention allows establishing the probability that a subject will suffer adverse effects in response to a nicotinic cholinergic receptor agonist drug-based therapy, wherein said adverse effects lead to the withdrawal of said therapy or to a reduction of the usual therapeutic dose of the drug used in said therapy. If said probability is high, for example, due to the presence of an allele associated with adverse effects in at least one of SNPs rs7930792, rs12423809, rs4251417, rs7146, rs477292, rs495491, rs3778151, rs9479757, rs763132, rs4474069 or rs11830355 (Table 1), then said subject may be an unsuitable candidate *a priori* for receiving said nicotinic cholinergic receptor agonist drug-based therapy, for example, varenicline-, cytisine- or dianicline-based therapy, among others, and must therefore receive an alternative therapy, or according to the case, receive a lower dose than the usual therapeutic dose of said drug. In contrast, if said probability is low, then said subject is a suitable candidate *a priori* for receiving a nicotinic cholinergic receptor agonist drug-based therapy.

This probabilistic model based on determining at least one of the alleles of one or more of the SNPs identified in the present invention, is a valid tool for predicting the probability that a nicotinic cholinergic receptor agonist drug-based therapy will cause the onset of adverse effects in a subject that lead to the withdrawal of said therapy or to a reduction of the usual therapeutic dose of the drug used in said therapy. In view of the results provided by this invention, the specialist (e.g., a doctor) can optimize the subject therapeutic care by choosing, from the start, the most suitable dose and/or indication depending on the probability of whether or not said nicotinic cholinergic receptor agonist drug-based therapy will cause adverse effects for the subject that lead to the withdrawal of said therapy or to a reduction of the usual therapeutic dose of the drug, and perform a close follow-up of the subject under treatment. Therefore, the methods and means provided by the present invention can help the doctors to select the therapy for treating a disease or pathology which can be treated with a nicotinic cholinergic receptor agonist drug, such that the possibility of withdrawing from therapy due to that reason is reduced, which aids therapeutic adherence, and furthermore the health expenditure is optimized. On the other hand, the adverse effects associated with the treatment with nicotinic cholinergic receptor agonist drugs in subjects the treatment of whom with that drug is ineffective, can be prevented.

### Method for predicting the safety of a nicotinic cholinergic receptor agonist drug-based therapy ("second method of the invention")

In another aspect, the invention relates to a method for predicting the safety of a nicotinic cholinergic receptor agonist drug-based therapy in a subject (hereinafter, "second method of the invention"), which comprises determining in a biological sample of said subject at least one allele of one or more of single-nucleotide polymorphisms (SNPs) rs7930792, rs12423809, rs4251417, rs7146, rs477292, rs495491, rs3778151, rs9479757, rs763132, rs4474069 or rs1183035, wherein
- the presence of at least one G allele of SNP rs7930792,
- the presence of at least one A allele of SNP rs12423809,
- the presence of at least one G allele of SNP rs4251417,
- the presence of at least one C allele of SNP rs7146,
- the presence of at least one G allele of SNP rs477292,
- the presence of at least one T allele of SNP rs495491,
- the presence of at least one T allele of SNP rs3778151,
- the presence of at least one G allele of SNP rs9479757,
- the presence of at least one G allele of SNP rs763132,
- the presence of at least one T allele of SNP rs4474069, and/or
- the presence of at least one C allele of SNP rs1183035,
is indicative that said nicotinic cholinergic receptor agonist drug-based therapy is safe at the usual therapeutic dose of the drug used in said therapy.

In an alternative embodiment of the second method of the invention, the invention also relates to a method for predicting the safety of a nicotinic cholinergic receptor agonist drug-based therapy to quit consuming tobacco in a subject, which comprises determining in a biological sample of said subject at least one allele of one or more of SNPs rs7930792, rs12423809, rs4251417, rs7146, rs477292, rs495491, rs3778151, rs9479757, rs763132, rs4474069 or rs1183035, wherein
- the presence of at least one C allele of SNP rs7930792,
- the presence of at least one C allele of SNP rs12423809,
- the presence of at least one A allele of SNP rs4251417,
- the presence of at least one T allele of SNP rs7146,
- the presence of at least one A allele of SNP rs477292,
- the presence of at least one C allele of SNP rs495491,
- the presence of at least one C allele of SNP rs3778151, and/or
- the presence of at least one A allele of SNP rs9479757,
is indicative that said nicotinic cholinergic receptor agonist drug-based therapy is not safe at the usual therapeutic dose of the drug used in said therapy,
or wherein
- the presence of at least one T allele of SNP rs763132,
- the presence of at least one C allele of SNP rs4474069, and/or
- the presence of at least one T allele of SNP rs1183035,
is indicative that said nicotinic cholinergic receptor agonist drug-based therapy is safe at a dose equal to or less than 1 mg/day of the drug used in said therapy.

As used herein, the expression "predicting the safety of a therapy" refers to the ability to predict whether or not there is an onset of adverse effects in a subject in response to a nicotinic cholinergic receptor agonist drug-based therapy, for example, varenicline-, cytisine- and dianicline-based therapy, among others, wherein said adverse effects lead to the withdrawal of said therapy or to a reduction of the usual therapeutic dose of the drug used in said therapy. The consideration that the nicotinic cholinergic receptor agonist drug-based therapy, for example, varenicline-, cytisine- and dianicline-based therapy, among others, is safe means that the subject substantially will not present adverse effects derived from following said therapy that lead to the withdrawal of said therapy or to a reduction of the usual therapeutic dose of the drug used in said therapy.

Like the methods for determining said SNPs and identifying the nucleotides present in the polymorphic sites of said SNPs, the characteristics of the biological sample, the subject, the SNPs (rs7930792, rs12423809, rs4251417, rs7146, rs477292, rs495491, rs3778151, rs9479757, rs763132, rs4474069 or rs1183035), as well as the characteristics of the polymorphic sites thereof, have already been mentioned above in relation to the first method of the invention; all this is incorporated in this method by reference.

According to the second method of the invention, at least one allele of one or more of SNPs rs7930792, rs12423809, rs4251417, rs7146, rs477292, rs495491, rs3778151, rs9479757, rs763132, rs4474069 and rs1183035, is determined in a biological sample of the subject under study, and the obtained results are correlated with the prediction of whether or not there is an onset of adverse effects in said subject in response to a nicotinic cholinergic receptor agonist drug-based therapy that lead to the withdrawal of said therapy or to a reduction of the usual therapeutic dose of the drug used in said therapy (prediction of the safety of a therapy). Non-limiting illustrative examples of nicotinic cholinergic receptor agonist drugs include cytisine, dianicline and varenicline. In a particular embodiment, said nicotinic cholinergic receptor agonist drug is varenicline.

Once at least one of the alleles of one or more of SNPs rs7930792, rs12423809, rs4251417, rs7146, rs477292, rs495491, rs3778151, rs9479757, rs763132, rs4474069 and rs1183035, is determined in a biological sample of said subject according to the second method of the invention, it is possible to predict the safety of a nicotinic cholinergic receptor agonist drug-based therapy in a subject. The prediction that said therapy will be safe or not safe in a subject can be carried out based on the presence or absence of the "alleles associated with the safety of the therapy" (i.e., based on the presence or absence of the nucleotides present in the polymorphic sites of said SNPs associated with a prediction that said nicotinic cholinergic receptor agonist drug-based therapy is safe or not safe) of said SNPs in the genome of the subject under study. Said alleles associated with the safety of the therapy, i.e., the presence of which is associated with a prediction that the nicotinic cholinergic receptor agonist drug-based therapy is safe, are listed in Table 2.

**Table 2**

| Alleles associated with the safety of a nicotinic cholinergic receptor agonist drug-based therapy ("alleles associated with the safety of the therapy") for the different SNPs identified in the present invention | |
|---|---|
| SNP | Allele associated with the safety of the therapy |
| rs7930792 | G |
| rs12423809 | A |
| rs4251417 | G |
| rs7146 | C |
| rs477292 | G |
| rs495491 | T |
| rs3778151 | T |
| rs9479757 | G |
| rs763132 | G |
| rs4474069 | T |
| rs1183035 | C |

In a particular embodiment of the second method of the invention:
- the presence of at least one G allele of SNP rs7930792,
- the presence of at least one A allele of SNP rs12423809,
- the presence of at least one G allele of SNP rs4251417,
- the presence of at least one C allele of SNP rs7146,
- the presence of at least one G allele of SNP rs477292,
- the presence of at least one T allele of SNP rs495491,
- the presence of at least one T allele of SNP rs3778151,
- the presence of at least one G allele of SNP rs9479757,
- the presence of at least one G allele of SNP rs763132,
- the presence of at least one T allele of SNP rs4474069, and/or
- the presence of at least one C allele of SNP rs1183035,
is indicative that said nicotinic cholinergic receptor agonist drug-based therapy is safe at the usual therapeutic dose of the drug used in said therapy.

Alternatively, in another particular embodiment of the alternative embodiment of the second method of the invention:
- the presence of at least one C allele of SNP rs7930792,
- the presence of at least one C allele of SNP rs12423809,
- the presence of at least one A allele of SNP rs4251417,
- the presence of at least one T allele of SNP rs7146,
- the presence of at least one A allele of SNP rs477292,
- the presence of at least one C allele of SNP rs495491,
- the presence of at least one C allele of SNP rs3778151, and/or
- the presence of at least one A allele of SNP rs9479757,
is indicative that said nicotinic cholinergic receptor agonist drug-based therapy is not safe at the usual therapeutic dose of the drug used in said therapy.

Alternatively, in another particular embodiment of the alternative embodiment of the second method of the invention:
- the presence of at least one T allele of SNP rs763132;
- the presence of at least one C allele of SNP rs4474069; and/or
- the presence of at least one T allele of SNP rs1183035;
is indicative that said nicotinic cholinergic receptor agonist drug-based therapy is safe at a dose equal to or less than 1 mg/day of the drug used in said therapy.

Likewise, in a particular embodiment of the second method of the invention:
- the presence of both G alleles of SNP rs7930792,
- the presence of both A alleles of SNP rs12423809,
- the presence of both G alleles of SNP rs4251417,
- the presence of both C alleles of SNP rs7146,
- the presence of both G alleles of SNP rs477292,
- the presence of both T alleles of SNP rs495491,
- the presence of both T alleles of SNP rs3778151,
- the presence of both G alleles of SNP rs9479757,
- the presence of both G alleles of SNP rs763132,
- the presence of both T alleles of SNP rs4474069, and/or
- the presence of both C alleles of SNP rs1183035,
is indicative that said nicotinic cholinergic receptor agonist drug-based therapy is safe at the usual therapeutic dose of the drug used in said therapy.

Alternatively, it can be established that:
- the presence of both C alleles of SNP rs7930792,
- the presence of both C alleles of SNP rs12423809,
- the presence of both A alleles of SNP rs4251417,
- the presence of both T alleles of SNP rs7146,
- the presence of both A alleles of SNP rs477292,
- the presence of both C alleles of SNP rs495491,
- the presence of both C alleles of SNP rs3778151, and/or
- the presence of both A alleles of SNP rs9479757,
is indicative that said nicotinic cholinergic receptor agonist drug-based therapy is not safe at the usual therapeutic dose of the drug used in said therapy.

Alternatively, it can be established that:
- the presence of both T alleles of SNP rs763132,
- the presence of both C alleles of SNP rs4474069, and/or
- the presence of both T alleles of SNP rs1183035,
is indicative that said nicotinic cholinergic receptor agonist drug-based therapy is safe at a dose equal to or less than 1 mg/day of the drug used in said therapy.

The second method of the invention allows predicting the safety of a nicotinic cholinergic receptor agonist drug-based therapy in a subject, such that if the result of said prediction is that the therapy is safe, then the subject can be treated with a nicotinic cholinergic receptor agonist drug, whereas, otherwise, if the result of said prediction is that the therapy is not safe, then the subject should not be treated with said drug or, depending on the case, doses of said nicotinic cholinergic receptor agonist drug less than the usual therapeutic dose should be used.

This predictive model based on determining at least one of the alleles of one or more of the SNPs identified in the present invention, is a valuable tool for predicting whether or not a nicotinic cholinergic receptor agonist drug-based therapy will be safe for a subject, i.e., if the administration of said drug to said subject will cause the onset of adverse effects that lead to the withdrawal of said therapy or to a reduction of the usual therapeutic dose of the drug used in said therapy. In view of the results provided by this invention, the specialist (e.g., a doctor) could optimize subject therapeutic care by choosing, from the start, the most suitable dose and/or indication depending on the probability of whether or not said nicotinic cholinergic receptor agonist drug-based therapy will be safe for the subject and perform a close follow-up of the subject under treatment.

In a particular embodiment, the methods and means provided by the present invention can help doctors select the therapy for quitting tobacco consumption that is the most suitable for a subject. In a particular embodiment, said nicotinic cholinergic receptor agonist drug is varenicline.

### Method for selecting a subject to follow a nicotinic cholinergic receptor agonist drug-based therapy (hereinafter, "third method of the invention")

In another aspect, the invention relates to a method for selecting a subject to follow a nicotinic cholinergic receptor agonist drug-based therapy (hereinafter, "third method of the invention"), which comprises determining in a biological sample of said subject at least one allele of one or more of single-nucleotide polymorphisms (SNPs) rs7930792, rs12423809, rs4251417, rs7146, rs477292, rs495491, rs3778151, rs9479757, rs763132, rs4474069 or rs1183035, wherein said subject is selected for said therapy if he/she has
- at least one G allele of SNP rs7930792,
- at least one A allele of SNP rs12423809,
- at least one G allele of SNP rs4251417,
- at least one C allele of SNP rs7146,
- at least one G allele of SNP rs477292,
- at least one T allele of SNP rs495491,
- at least one T allele of SNP rs3778151,
- at least one G allele of SNP rs9479757,
- at least one G allele of SNP rs763132,
- at least one T allele of SNP rs4474069, and/or
- at least one C allele of SNP rs1183035.

In a particular embodiment of the third method of the invention, a subject is selected for a nicotinic cholinergic receptor agonist drug-based therapy when said subject has
- both G alleles of SNP rs7930792,
- both A alleles of SNP rs12423809,
- both G alleles of SNP rs4251417,
- both C alleles of SNP rs7146,
- both G alleles of SNP rs477292,
- both T alleles of SNP rs495491,
- both T alleles of SNP rs3778151,
- both G alleles of SNP rs9479757,
- both G alleles of SNP rs763132,
- both T alleles of SNP rs4474069, and/or
- both C alleles of SNP rs1183035.

Like the methods for determining said SNPs and identifying the nucleotides present in the polymorphic sites of said SNPs, the characteristics of the biological sample, the subject, the SNPs (rs7930792, rs12423809, rs4251417, rs7146, rs477292, rs495491, rs3778151, rs9479757, rs763132, rs4474069, rs1183035) as well as the characteristics of the polymorphic sites thereof, have already been mentioned above in relation to the first method of the invention; all this is incorporated in this method by reference.

According to the third method of the invention, at least one allele of one or more of SNPs rs7930792, rs12423809, rs4251417, rs7146, rs477292, rs495491, rs3778151 and rs9479757, rs763132, rs4474069, rs1183035, is determined in a biological sample of the subject under study, and the obtained results are correlated with the possibility of selecting a subject so that he/she receives a nicotinic cholinergic receptor agonist drug-based therapy. Non-limiting illustrative examples of nicotinic cholinergic receptor agonist drugs include cytisine, dianicline and varenicline. In a particular embodiment, said nicotinic cholinergic receptor agonist drug is varenicline. In a particular embodiment, the nicotinic cholinergic receptor agonist drug-based therapy is intended for the treatment of a chemical addiction or dependence, particularly a nicotine or tobacco addiction (or dependence). In another particular embodiment, the nicotinic cholinergic receptor agonist drug-based therapy is intended for the treatment of a disease or pathology which can be treated with a nicotinic cholinergic receptor agonist drug different from a chemical addiction or dependence.

Once at least one of the alleles of one or more of SNPs rs7930792, rs12423809, rs4251417, rs7146, rs477292, rs495491, rs3778151 and rs9479757 rs763132, rs4474069, rs1183035, is determined in a biological sample of said subject, according to the third method of the invention, it is possible to select a subject so that he/she receives or does not receive a nicotinic cholinergic receptor agonist drug-based therapy. The selection of the subject can be carried out based on the presence or absence of the "alleles associated with the safety of the therapy" of said SNPs in the genome of the subject under study. Said alleles associated with the safety of the nicotinic cholinergic receptor agonist drug-based therapy are mentioned in Table 2.

The third method of the invention allows selecting a subject so that he/she receives a nicotinic cholinergic receptor agonist drug-based therapy such that, if the subject is selected, then said subject can be treated with a nicotinic cholinergic receptor agonist drug, whereas, otherwise, if the subject is not selected for said therapy, then the subject should not be treated with a nicotinic cholinergic receptor agonist drug, or, depending on the case (i.e., in view of the alleles of the SNPs), doses less than the usual therapeutic dose of said drug should be used.

This selective model based on determining at least one of the alleles of one or more of the SNPs identified in the present invention, is a valuable tool for selecting subjects who can be subjected to a nicotinic cholinergic receptor agonist drug-based therapy. In view of the results provided by this invention, the specialist (e.g., a doctor) could select the subject to be subjected to a nicotinic cholinergic receptor agonist drug-based therapy and perform a close follow-up of the subjects subjected to said therapy. Non-limiting illustrative examples of nicotinic cholinergic receptor agonist drugs include varenicline, cytisine or dianicline, or any other drug with a similar mechanism of action, preferably varenicline.

### Method for selecting a therapy for a subject in need of therapy (hereinafter, "fourth method of the invention")

In another aspect, the invention relates to a method for selecting a therapy for a subject suffering a disease or pathology which can be treated by means of a pharmacological therapy (hereinafter, "fourth method of the invention"), which comprises determining in a biological sample of said subject at least one allele of one or more of single-nucleotide polymorphisms (SNPs) rs7930792, rs12423809, rs4251417, rs7146, rs477292, rs495491, rs3778151, rs9479757, rs763132, rs4474069 or rs1183035, wherein a nicotinic cholinergic receptor agonist drug-based therapy is selected if said subject has
- at least one G allele of SNP rs7930792,
- at least one A allele of SNP rs12423809,
- at least one G allele of SNP rs4251417,
- at least one C allele of SNP rs7146,
- at least one G allele of SNP rs477292,
- at least one T allele of SNP rs495491,
- at least one T allele of SNP rs3778151,
- at least one G allele of SNP rs9479757,
- at least one G allele of SNP rs763132,
- at least one T allele of SNP rs4474069, and/or
- at least one C allele of SNP rs1183035.

In a particular embodiment of the fourth method of the invention, a nicotinic cholinergic receptor agonist drug-based therapy is selected when said subject has
- both G alleles of SNP rs7930792,
- both A alleles of SNP rs12423809,
- both G alleles of SNP rs4251417,
- both C alleles of SNP rs7146,
- both G alleles of SNP rs477292,
- both T alleles of SNP rs495491,
- both T alleles of SNP rs3778151,
- both G alleles of SNP rs9479757,
- both G alleles of SNP rs763132,
- both alleles of SNP rs4474069, and/or
- both alleles of SNP rs1183035.

Like the methods for determining said SNPs and identifying the nucleotides present in the polymorphic sites of said SNPs, the characteristics of the biological sample, the subject, SNPs rs7930792, rs12423809, rs4251417, rs7146, rs477292, rs495491, rs3778151, rs9479757, rs763132, rs4474069 and rs1183035, as well as the characteristics of the polymorphic sites thereof, have been mentioned above in relation to the first method of the invention; all this is incorporated in this method by reference.

According to the fourth method of the invention, at least one allele of one or more of SNPs rs7930792, rs12423809, rs4251417, rs7146, rs477292, rs495491, rs3778151, rs9479757, rs763132, rs4474069 and rs1183035, is determined in a biological sample of the subject under study, and the obtained results are correlated with the possibility of selecting a therapy for a subject in need of receiving therapy. In a particular embodiment, said therapy is intended for the treatment of a chemical addiction or dependence, particularly a nicotine or tobacco addiction (or dependence).

Once at least one of the alleles of one or more of SNPs rs7930792, rs12423809, rs4251417, rs7146, rs477292, rs495491, rs3778151, rs9479757, rs763132, rs4474069 and rs1183035, is determined in a biological sample of said subject, according to the fourth method of the invention, it is possible to select (or not) a nicotinic cholinergic receptor agonist drug-based therapy to be applied to the subject. The selection of said therapy can be carried out based on the presence or absence of the "alleles associated with the safety of the therapy" of said SNPs in the genome of the subject under study. Said alleles associated with the safety of the nicotinic cholinergic receptor agonist drug-based therapy are mentioned in Table 2.

In a particular embodiment, the fourth method of the invention allows selecting a nicotinic cholinergic receptor agonist drug-based therapy for treating a chemical addiction or dependence, particularly a nicotine or tobacco addiction (or dependence), such that that therapy is selected from among other therapies for treating said addiction or dependence when the genotype of the subject to be subjected to the therapy has the characteristics mentioned above, whereas, otherwise, if the subject does not show those characteristics, then a therapy for treating said chemical addiction or dependence that is not a nicotinic cholinergic receptor agonist drug-based therapy would have to be selected.

This selective model based on determining at least one of the alleles of one or more of the SNPs identified in the present invention, is a valuable tool for selecting a therapy for a subject in need of therapy. In view of the results provided by this invention, the specialist (e.g., a doctor) can select the therapy to be administered to the subject, specifically a nicotinic cholinergic receptor agonist drug-based therapy, and perform a close follow-up of the subjects subjected to said therapy. Non-limiting illustrative examples of nicotinic cholinergic receptor agonist drugs include varenicline, cytisine or dianicline, or any other drug with a similar mechanism of action, preferably varenicline.

### Kits of the Invention

The invention also contemplates the preparation of kits for use according to the methods of the present invention. Therefore, in another aspect the invention relates to a kit, hereinafter "kit of the invention", consisting of the reagents necessary for determining the presence of at least one allele of each of the SNPs rs7930792, rs12423809, rs4251417, rs7146, rs477292, rs495491, rs3778151, rs9479757, rs763132, rs4474069 and rs1183035, and/or the reagents for determining the nucleotide present in the polymorphic site of said SNPs.

The kit of the invention consists of the reagents necessary for determining the presence of all the 11 SNPs rs7930792, rs12423809, rs4251417, rs7146, rs477292, rs495491, rs3778151, rs9479757, rs763132, rs4474069, rs1183035, and/or the reagent necessary for determining the nucleotide present in said 11 SNPs.

The kit of the invention includes reagents for use according to the present invention in suitable containers and packaging materials, including tubes, vials, and shrink-wrapped and blow-molded packaging techniques. Materials suitable for inclusion in a kit according to the present invention comprise one or more of the following: specific PCR primer pairs (oligonucleotides) which hybridize with the DNA or cDNA sequence domains flanking the SNPs of interest (SNPs rs7930792, rs12423809, rs4251417, rs7146, rs477292, rs495491, rs3778151, rs9479757, rs763132, rs4474069, rs1183035); reagents capable of amplifying a specific sequence domain in either the gDNA or cDNA without having to perform PCR; reagents necessary for discriminating between the different alleles in the sequence of the domains amplified by PCR and non-PCR amplification (for example, restriction endonucleases, oligonucleotides preferably hybridizing with an allele of the polymorphism, including those modified to contain enzymes or fluorescent chemical groups that amplify the signal of oligonucleotides and make discrimination of alleles more robust); reagents necessary for physically separating the products derived from the different alleles (for example, agarose or polyacrylamide and a buffer to be used in electrophoresis, HPLC columns, SSCP gels, formamide gels or a matrix support for MALDI-TOF), etc.

The kits of the invention consist of allele-specific or polymorphism-specific oligonucleotides or oligonucleotide pairs, where each allele-specific or polymorphism-specific oligonucleotide pair targets one of the polymorphism mentioned in this document, i.e., SNPs rs7930792, rs12423809, rs4251417, rs7146, rs477292, rs495491, rs3778151, rs9479757, rs763132, rs4474069, rs1183035. The person skilled in the art will understand that in this context the term "targets" means an oligonucleotide or an oligonucleotide pair which is capable of identifying the allele present in a certain SNP.

The present invention contemplates kits that comprise a set of probes, comprising a plurality of oligonucleotide probes interrogating for all SNPs of the group consisting of SNPs rs7930792, rs12423809, rs4251417, rs7146, rs477292, rs495491, rs3778151, rs9479757, rs763132, rs4474069, rs1183035.

In a particular embodiment, the kit consists of polymorphism-specific or allele-specific oligonucleotides or oligonucleotide pairs targeting all the SNPs rs7930792, rs12423809, rs4251417, rs7146, rs477292, rs495491, rs3778151, rs9479757, rs763132, rs4474069, rs1183035.

The kit of the invention allows predicting the safety of a nicotinic cholinergic receptor agonist drug-based therapy. Therefore, in another aspect the invention relates to the use of the kit of the invention for:
- determining the probability that a subject will suffer adverse effects in response to a nicotinic cholinergic receptor agonist drug-based therapy, wherein said adverse effects lead to the withdrawal of said therapy or to a reduction of the usual therapeutic dose of the drug used in said therapy; or for
- predicting the safety of a nicotinic cholinergic receptor agonist drug-based therapy in a subject; or for
- selecting a subject for a nicotinic cholinergic receptor agonist drug-based therapy; or for
- selecting a therapy to be administered to a subject in need of therapy.

### Uses of the Invention

In another aspect, the invention relates to the use of a single-nucleotide polymorphism (SNP) for:
- determining the probability that a subject will suffer adverse effects in response to a nicotinic cholinergic receptor agonist drug-based therapy, wherein said adverse effects lead to the withdrawal of said therapy or to a reduction of the usual therapeutic dose of the drug used in said therapy; or for
- predicting the safety of a nicotinic cholinergic receptor agonist drug-based therapy in a subject; or for
- selecting a subject for a nicotinic cholinergic receptor agonist drug-based therapy; or for
- selecting a therapy to be administered to a subject in need of therapy,
wherein said SNP is selected from the group consisting of rs7930792, rs12423809, rs4251417, rs7146, rs477292, rs495491, rs3778151, rs9479757, rs763132, rs4474069 or rs1183035 and any combination thereof.

In a particular embodiment, said SNP is selected from the group consisting of SNPs rs7930792, rs12423809, rs4251417, rs7146, rs477292, rs495491, rs3778151, rs9479757, or any combination of said SNPs,. In another particular embodiment, said SNP is selected from the group consisting of SNPs rs763132, rs4474069, rs1183035, or any combination of said SNPs.

In another aspect, the invention relates to a nicotinic cholinergic receptor agonist drug-based therapy for use in the treatment of a subject in need of therapy, wherein said subject is selected by means of the third method of the invention. In a particular embodiment, said nicotinic cholinergic receptor agonist drug is selected from varenicline, cytisine and dianicline, preferably varenicline.

In another aspect, the invention relates to a nicotinic cholinergic receptor agonist drug-based therapy for treating a disease or pathology which can be treated with a nicotinic cholinergic receptor agonist drug, for use in the treatment of a subject in need of said therapy for treating a disease or pathology which can be treated with a nicotinic cholinergic receptor agonist drug, wherein said subject is selected by means of the third method of the invention. In a particular embodiment, said nicotinic cholinergic receptor agonist drug is selected from varenicline, cytisine and dianicline, preferably varenicline. In another particular embodiment, said disease or pathology which can be treated with a nicotinic cholinergic receptor agonist drug is a chemical addiction or dependence, particularly a nicotine or tobacco addiction (or dependence). In another particular embodiment, said disease or pathology which can be treated with a nicotinic cholinergic receptor agonist drug is a disease or pathology which can be treated with a nicotinic cholinergic receptor agonist drug different from a chemical addiction or dependence.

The following example serves to illustrate the invention and must not be considered as limiting the scope thereof.

### EXAMPLE 1

### Identification of SNPs associated with safety in quitting tobacco consumption in subjects treated with varenicline

### 1. MATERIALS AND METHODS

### Study groups and parameters

In a study conducted on 807 patients from the Tobacco Addiction Units of 22 hospitals and health centers in Spain and Portugal, more than 300 SNPs relating to the relevant pathways with respect to the efficacy of the 3 first-line pharmacological tobacco cessation treatments were assessed: nicotine replacement therapy (NRT), bupropion and varenicline. The safety was assessed after three months of abstinence from tobacco consumption in relation to the presence of adverse effects which determined the reduction or withdrawal of the initially applied treatment.

The analysis process was carried out after applying the usual checks for correcting data entry errors. The status of the subjects was first characterized either as "cases" or as "controls", depending on the safety results obtained after 3 months of follow-up after the establishment of the chosen tobacco cessation treatment. Those individuals in whom (a) it was necessary to reduce the dose of the chosen treatment due to the presence of treatment-related adverse effects, or (b) it was necessary to suspend or change the treatment due to intolerance to same according to the assessment of his/her doctor, were considered as "cases". The different statistical analysis models thus provided information about the safety associated with the presence of a certain genetic pattern.

To assess the possibility of confounding biases as a result of the mixed population analysis, and also as a control for genotyping errors, the Hardy-Weinberg disequilibrium test was performed both in the "cases" and in the corresponding "controls". The result of this test was assessed before analyzing the results obtained in the various logistic regression models which were used to assess the association between the specific SNPs and the safety results of the prescribed treatments. The following penetrance models were used in the logistic regression models: dominant, recessive, additive, over-dominant and log-additive. The statistical analyses were performed both with the allelic frequency and with the genotypic frequency, the latter being those which provide the most information about the associations to be replicated. All the results were presented as odds-ratios (OR) with the corresponding 95% confidence intervals and statistical significance value thereof.

Finally, the discriminating power of the different genetic indicators (SNPs) analyzed with respect to the safety results was assessed by means of operator-receptor curves and area under the curve analysis. The final purpose of this analysis strategy was to enable estimating the probability of adverse effects that a subject with a certain genotypic profile who starts a tobacco cessation treatment (particularly with varenicline) may have and adjusting and monitoring his/her treatment based on this information.

### Genotyping

The nucleic acid samples can be genotyped to determine the alleles present in a certain gene region of interest, for example, the position of a certain SNP, by means of methods well known in the state of the art. The candidate SNPs were identified by means of 3 different strategies mainly intended for including SNPs with a potential functional impact on protein structure and/or gene expression, as well as from the information about common variations along a given gene.

According to a first strategy, coding, non-synonymous SNPs (nsSNPs) and SNPs located in potential regions (in the 5' upstream direction, 5'-UTR, coding region, splicing sites, first intron, 3'-UTR and 3' downstream) were sought. The SNPs were sought from 10 kilobases (kb) in the 5' upstream direction from the start codon to 10 kbs 3' downstream from the stop codon for a given gene. The SNPs included in this group were those with a possible functional effect, specifically those which alter potential transcription factor binding sites such as those which can be predicted by means of using the Pupas View software (Conde et al. 2005, Nucleic Acid Res 33: W501-5), and SNPs located in interspecies conserved sequences, as determined by means of using the UCSC Genome Browser Database (http://genome.ucsc.edu/index.html).

In a second strategy, several target SNPs were selected. The main purpose of this selection was to identify a set of SNPs targeting almost all the known SNPs in a gene, including those located in potential regulatory regions which did not show any evidence of functionality. In this study, the target SNPs were defined as common SNPs with an estimated r2 greater than 0.8 (r2 > 0.8) with other SNPs in subjects of European ancestry (CEU) in the HapMap database (Tagger, Haploview 3.2) (Barrett et al. 2005 Bioinformatics 21(2):263-265).

Finally, in the third strategy SNPs previously described in the literature were considered.

In all cases, only those SNPs with a described minor allele frequency of less than 5% (MAF ≥ 0.05) in the Caucasian populations of HapMap-CEU and PERLEGEN-EUR-Panel North America (ENSEMBL genome browser v. 3.3-3.6; www.ensembl.org) were selected for the purpose of assuring informative SNPs. All the selected SNPs were filtered by means of Illumina technology criteria (score ≥ 0.6 or Goldengate Validated Status, Illumina Inc., San Diego, USA). The genotyping of the SNPs was carried out in the National Genotyping Center (Centro Nacional de Genotipado) (CeGen), Spain, using the Illumina Bead Array system (Illumina Inc., San Diego; USA).

Illumina Bead Array technology is based on 3 µm beads which self-aggregate in microwells on one of the substrates: fiber optic bundles or planar silica surfaces. When the beads are randomly assembled on one of these 2 substrates, they are arranged with a uniform separation of about 5.7 mm. Each bead is covered by hundreds of thousands of copies of a specific oligonucleotide which acts by capturing the sequences in one of the Illumina assays. On average, each type of bead is represented 30 times per sample, allowing a high degree of precision. The GoldenGate Genotyping assay was selected. In this approach, during the liquid phase, allele-specific oligonucleotides (ASO) hybridize with gDNA, elongate and ligate with a locus-specific oligonucleotide (LSO). PCR was carried out using universal primers. The products of the multiplex reaction were hybridized for detection and analysis in a universal Sentrix Array. The results obtained in duplicate blood DNA samples were consistent with all the SNPs genotyped by this method. The DNA was quantified in the National Genotyping Center (Centro Nacional de Genotipado) (CeGen) by means of the PicoGreen technique and diluted to a final concentration of 50 ng/ml. With this technique, the DNA concentration was determined by means of a fluorescent marker (PicoGreen®, Molecular Probes) which binds to a doublestranded DNA and subsequent quantification in a fluorometer.

### 2. RESULTS

For the purpose of determining the pharmacogenetic profile associated with the safety of a pharmacological smoking cessation treatment, the results obtained in terms of abstinence from tobacco consumption after 3 months were evaluated in relation to the presence of adverse effects which determined the reduction or withdrawal of the initially applied treatment. The subjects were divided into three groups which were treated with NRT, bupropion or varenicline.

It is an observational study with the intervention of 21 Tobacco Addiction Units from the public health services in Spain and Portugal. A total of 807 subjects were invited to participate in the study, 798 of whom provided their sociodemographic and clinical data taking a test with 144 items. They also provided a biological sample for subsequent genotyping. 483 individuals were treated with varenicline. The information of both the phenotypic and genotypic variables was collected for 479 patients treated with varenicline before establishing the pharmacological treatment. The general description of the analyzed population is shown in Table 3.

**Table 3**

| Characteristics of the evaluated sample and sample under treatment with varenicline | | |
|---|---|---|
| Variables | Mean (SD) | N (%) |
| Sex: | | |
| Men | | 241 (49.9) |
| Women | | 242 (48.3) |
| Age (years) | 46.2 (10.8) | |
| Cigarette consumption/day | 23.9 (10.9) | |
| Age of starting tobacco consumption (years) | 15.5 (3.6) | |
| Age of starting daily consumption (years) | 18.1 (4.5) | |
| Number of attempts to stop smoking | 2.8 (7.1) | |
| Method used in the previous attempts: | | |
| NRT | | 115 (31.1) |
| Bupropion/varenicline | | 36 (9.7) |
| Without drugs | | 219 (5.2) |
| Months since the last attempt to stop smoking | 41.5 (86.4) | |
| Maximum time of abstinence achieved (months) | 29.0 (80.1) | |

| | | |
|---|---|---|
| SD: standard deviation; NRT: nicotine replacement therapy | | |

The clinical results were linked to the safety of the pharmacological treatments. The safety was measured after 3 months with the criteria for suspending the treatment due to adverse effects and for reducing the dose as a result of the adverse effects.

Eighty candidate genes were selected based on their biological relevance and on the literature search, and based on the researchers' assumptions about the candidate genes. In the samples, 384 SNPs, insertion/deletion and tandem repetitions, were analyzed. The selection of the SNP for each gene was based on the following criteria:
1. target SNPs, r2 > 0.98 with other SNPs in HapMap individuals of European ancestry (CEU) (The International HapMap Project, 2003) (Tagger, Haploview 3.2).
2. SNPs located in regions which can have an influence on gene function (upstream, 5'UTR, coding region, splicing sites, intron 1, 3'UTR and downstream).
3. SNPs previously associated with the dependence and cessation of tobacco consumption.

The results obtained clearly showed the association between SNPs and the safety of the smoking cessation treatment only for those genes showing a p-value of less than 0.01. The genetic results are presented with their corresponding ORs (odds ratios), precision (95% confidence intervals) and the corresponding p-value. Thirteen SNPs associated with varenicline efficacy which are shown in Tables 4-7 were obtained.

The results which are shown below show the adjustment in the univariate model including the genotype as an explanatory variable. The explanatory power of this model was contrasted with the null model (without explanatory variable) by means of the likelihood-ratio test for nested models. A significant p-value indicates the need to explain the result by means of the univariate model including the genotype.

**Table 4**

| Univariate results (genotype) of the safety of a treatment with varenicline for tobacco cessation - treatment suspension due to adverse effects | | | | | | | |
|---|---|---|---|---|---|---|---|
| rs7930792 (Chr 11; UBASH3B) | | | | | | | |
| Result | Genotype | | | | P-value | AIC | AUC |
| | C/C | | C/G | G/G | | | |
| 1 | 27 | | 18 | 2 | | | |
| 0 | 158 | | 81 | 55 | | | |
| OR | 1.00 | | 0.58 | 0.21 | 0.0256 | 297.9 | |
| Linear OR | 0.52 | | | | 0.0079 | 296.2 | 0.6037 |
| | | | | | | | |

| Result | Alleles | | | | OR (95% CI) | P-value | |
|---|---|---|---|---|---|---|---|
| | C | | G | | | | |
| 1 | 72 | | 22 | | | | |
| 0 | 497 | | 291 | | | | |
| Total | 569 | | 313 | | 0.52 (0.32 - 0.86) | 0.011 | |
| | | | | | | | |
| Comments | | The C/G and G/G genotypes are linked to an increased safety, the G allele being the most important factor in explaining the safety of the treatment. | | | | | |
| | | | | | | | |
| | | Figure 1 shows the ROC curve corresponding to the univariate model in relation to SNP rs7930792. | | | | | |
| | | | | | | | |
| rs12423809 (Chr 12; C12orf36) | | | | | | | |

| Result | Genotype | | | | P-value | AIC | AUC |
|---|---|---|---|---|---|---|---|
| | A/A | | A/C | C/C | | | |
| 1 | 13 | | 21 | 13 | | | |
| 0 | 173 | | 176 | 49 | | | |
| OR | 1.00 | | 1.59 | 3.53 | 0.0141 | 297.6 | |
| Linear OR | 1.87 | | | | 0.0041 | 295.9 | 0.6127 |
| | | | | | | | |
| Result | Alleles | | | | OR (95% CI) | P-value | |
| | A | | C | | | | |
| 1 | 47 | | 47 | | | | |
| 0 | 522 | | 274 | | | | |
| Total | 569 | | 321 | | 1.91 (1.24 - 2.93) | 0.003 | |
| | | | | | | | |
| Comments | | The A/C and A/A genotypes are linked to an increased safety, the A allele being the most important factor in explaining the safety of the treatment. | | | | | |
| | | | | | | | |
| | | Figure 2 shows the ROC curve corresponding to the univariate model in relation to SNP rs12423809. | | | | | |
| | | | | | | | |
| rs4251417 (Chr 17; SLC6A4) | | | | | | | |

| Result | Genotype | | | | P-value | AIC | AUC |
|---|---|---|---|---|---|---|---|
| | G/G | | A/G | A/A | | | |
| 1 | 34 | | 11 | 2 | | | |
| 0 | 340 | | 56 | 1 | | | |
| OR | 1.00 | | 1.96 | 20.0 | 0.0149 | 297.5 | |
| Linear OR | 2.43 | | | | 0.0084 | 297.0 | 0.5692 |
| | | | | | | | |

| Result | Alleles | | | | OR (95% CI) | P -value | |
|---|---|---|---|---|---|---|---|
| | G | | A | | | | |
| 1 | 79 | | 15 | | | | |
| 0 | 736 | | 58 | | | | |
| Total | 815 | | 73 | | 2.41 (1.30 - 4.50) | 0.005 | |
| | | | | | | | |
| Comments | | The A/G and G/G genotypes are linked to an increased safety, the G allele being the most important factor in explaining the safety of the treatment. | | | | | |
| | | | | | | | |
| | | Figure 3 shows the ROC curve corresponding to the univariate model in relation to SNP rs4251417. | | | | | |
| | | | | | | | |
| rs7146 (Chr 19; C19orf6) | | | | | | | |

| Result | Genotype | | | | P-value | AIC | AUC |
|---|---|---|---|---|---|---|---|
| | C/C | | C/T | T/T | | | |
| 1 | 15 | | 23 | 9 | | | |
| 0 | 189 | | 180 | 28 | | | |
| OR | 1.00 | | 1.61 | 4.05 | 0.0162 | 297.7 | |
| Linear OR | 1.93 | | | | 0.0053 | 296.2 | 0.6046 |
| | | | | | | | |

| Result | Alleles | | | | OR (95% CI) | P-value | |
|---|---|---|---|---|---|---|---|
| | C | | T | | | | |
| 1 | 53 | | 41 | | | | |
| 0 | 558 | | 236 | | | | |
| Total | 611 | | 277 | | 1.83 (1.18 - 2.83) | 0 .007 | |
| | | | | | | | |
| Comments | | The C/T and C/C genotypes are linked to an increased safety, the C allele being the most important factor in explaining the safety of the treatment. | | | | | |
| | | | | | | | |
| | | Figure 4 shows the ROC curve corresponding to the univariate model in relation to SNP rs7146. | | | | | |
| | | | | | | | |
| rs477292 (Chr 6; OPRM1) | | | | | | | |

| Result | Genotype | | | | P-value | AIC | AUC |
|---|---|---|---|---|---|---|---|
| | G/G | | A/G | A/A | | | |
| 1 | 20 | | 23 | 4 | | | |
| 0 | 248 | | 134 | 14 | | | |
| OR | 1.00 | | 2.13 | 3.54 | 0.0229 | 298.2 | |
| Linear | 1.99 | | | | 0.0064 | 296.3 | 0.6061 |
| OR | | | | | | | |
| | | | | | | | |

| Result | Alleles | | | | OR (95% CI) | P-value | |
|---|---|---|---|---|---|---|---|
| | G | | A | | | | |
| 1 | 63 | | 31 | | | | |
| 0 | 630 | | 162 | | | | |
| Total | 693 | | 193 | | 1.91 (1.20 - 3.04) | 0.006 | |
| | | | | | | | |
| Comments | | The A/G and G/G genotypes are linked to an increased safety, the G allele being the most important factor in explaining the safety of the treatment. | | | | | |
| | | | | | | | |
| | | Figure 5 shows the ROC curve corresponding to the univariate model in relation to SNP rs477292. | | | | | |
| | | | | | | | |
| rs495491 (Chr 6; OPRM1) | | | | | | | |

| Result | Genotype | | | | P-value | AIC | AUC |
|---|---|---|---|---|---|---|---|
| | T/T | | C/T | C/C | | | |
| 1 | 15 | | 27 | 5 | | | |
| 0 | 228 | | 148 | 20 | | | |
| OR | 1.00 | | 2.77 | 3.80 | 0.0031 | 294.1 | |
| Linear OR | 2.19 | | | | 0.0012 | 293.2 | 0.6337 |
| | | | | | | | |

| Result | Alleles | | | | OR (95% CI) | P-value | |
|---|---|---|---|---|---|---|---|
| | T | | C | | | | |
| 1 | 57 | | 37 | | | | |
| 0 | 604 | | 188 | | | | |
| Total | 661 | | 225 | | 2.08 (1.34 - 3.25) | 0.001 | |
| | | | | | | | |
| Comments | | The C/T a nd T/T genotypes are link ed to an increased safety, the T allele being the most important factor in explaining the safety of the treatment. | | | | | |
| | | | | | | | |
| | | Figure 6 shows the ROC curve corresponding to the univariate model in relation to SNP rs495491. | | | | | |
| | | | | | | | |
| rs3778151 (Chr 6; OPRM1) | | | | | | | |

| Result | Genotype | | | | P-value | AIC | AUC |
|---|---|---|---|---|---|---|---|
| | T/T | | C/T | C/C | | | |
| 1 | 22 | | 19 | 4 | | | |
| 0 | 280 | | 107 | 6 | | | |
| OR | 1.00 | | 2.26 | 8.48 | 0.0024 | 283.9 | |
| Linear OR | 2.55 | | | | 0.0006 | 282.3 | 0.6207 |
| | | | | | | | |

| Result | Alleles | | | | OR (95% CI) | P-value | |
|---|---|---|---|---|---|---|---|
| | T | | C | | | | |
| 1 | 63 | | 27 | | | | |
| 0 | 667 | | 119 | | | | |
| Total | 730 | | 146 | | 2.40 (1.47 - 3.93) | 0. 0003 | |
| | | | | | | | |
| Comments | | The C/T and T/T genotypes are linked to an increased safety, the T allele being the most important factor in explaining the safety of the treatment. | | | | | |
| | | | | | | | |
| | | Figure 7 shows the ROC curve corresponding to the univariate model in relation to SNP rs3778151. | | | | | |
| | | | | | | | |
| rs9479757 (Chr 6; OPRM1) | | | | | | | |

| Result | Genotype | | | | P-value | AIC | AUC |
|---|---|---|---|---|---|---|---|
| | G/G | | A/G | A/A | | | |
| 1 | 27 | | 18 | 2 | | | |
| 0 | 332 | | 64 | 2 | | | |
| OR | 1.00 | | 3.46 | 12.30 | 0.0002 | 289.5 | |
| Linear OR | 3.47 | | | | 0.00004 | 287.5 | 0.6323 |
| | | | | | | | |

| Result | Alleles | | | | OR (95% CI) | P-value | |
|---|---|---|---|---|---|---|---|
| | G | | A | | | | |
| 1 | 72 | | 22 | | | | |
| 0 | 728 | | 68 | | | | |
| Total | 800 | | 90 | | 3.27 (1.91 - 5.60) | 6.2 x 10⁻⁶ | |
| | | | | | | | |
| Comments | | The A/G and G/G genotypes are linked to an increased safety, the G allele being the most important factor in explaining the safety of the treatment. | | | | | |
| | | | | | | | |
| | | Figure 8 shows the ROC curve corresponding to the univariate model in relation to SNP rs9479757. | | | | | |

Likewise, the authors of the present invention evaluated the safety prediction capability in response to a varenicline-based pharmacological treatment of the set of SNPs consisting of rs7930792, rs12423809, rs4251417, rs7146, rs477292, rs495491 and rs9479757 (rs3778151 is eliminated due to co-linearity problems since it was highly and directly correlated with other SNPs already included in the model) with respect to the safety predictive value of each of said SNPs analyzed in an isolated manner, showing that the analysis of the set of mentioned SNPs improve the prediction reliability (AUC = 0.7779) with respect to the prediction capability of each of the SNPs previously analyzed in an individual manner (AUC (rs7930792) = 0.6037; AUC (rs12423809) = 0.6127; AUC (rs4251417) = 0.5692; AUC (rs7146) = 0.6046; AUC (rs477292) = 0.6061; AUC (rs495491) = 0.6337; AUC (rs3778151) = 0.6207; AUC (rs9479757) = 0.6323).

**Table 5**

| Univariate results (genotype) of the safety of a treatment with varenicline for tobacco cessation - dose reduction due to adverse effects | | | | | | |
|---|---|---|---|---|---|---|
| rs763132 (Chr 6; ARHGAP18) | | | | | | |
| Result | Genotype | | | P-value | AIC | AUC |
| | T/T | G/T | G/G | | | |
| 1 | 36 | 21 | 2 | | | |
| 0 | 148 | 175 | 61 | | | |
| OR | 1.00 | 0.49 | 0.13 | 0.0007 | 339.1 | |
| Linear OR | 0.44 | | | 0.0002 | 337.6 | 0.6329 |
| | | | | | | |

| Result | Alleles | | | OR (95% CI) | P-value | |
|---|---|---|---|---|---|---|
| | T | G | | | | |
| 1 | 93 | 25 | | | | |
| 0 | 471 | 297 | | | | |
| Total | 564 | 322 | | 0.43 (0.27 - 0.68) | 0.0002 | |
| | | | | | | |
| Comments | The G/T and G/G genotypes are linked to an increased safety, the G allele being the most important factor in explaining the safety of the treatment. | | | | | |
| | | | | | | |
| | Figure 9 shows the ROC curve corresponding to the univariate model in relation to SNP rs763132. | | | | | |
| | | | | | | |

| rs4474069 (Chr 9; RAPGEF1) | | | | | | |
|---|---|---|---|---|---|---|
| Result | Genotype | | | P-value | AIC | AUC |
| | C/C | C/T | T/T | | | |
| 1 | 25 | 32 | 2 | | | |
| 0 | 249 | 119 | 15 | | | |
| OR | 1.00 | 2. 68 | 1.33 | 0.0029 | 341.7 | |
| Linear OR | 1.87 | | | 0.0070 | 344.1 | 0.6078 |
| | | | | | | |

| Result | Alleles | | | OR (95% CI) | P-value | |
|---|---|---|---|---|---|---|
| | C | T | | | | |
| 1 | 82 | 36 | | | | |
| 0 | 617 | 149 | | | | |
| Total | 699 | 185 | | 1.82 (1.18 - 2.80) | | 0.007 |
| | | | | | | |
| Comments | The C/T and T/T genotypes are linked to an increased safety, the C allele being the most important factor in explaining the safety of the treatment. | | | | | |
| | | | | | | |
| | Figure 10 shows the ROC curve corresponding to the univariate model in relation to SNP rs4474069. | | | | | |
| | | | | | | |

| rs1183035 (Chr X; MAOB) | | | | | | |
|---|---|---|---|---|---|---|
| Result | Genotype | | | P-value | AIC | AUC |
| | T/T | C/T | C/C | | | |
| 1 | 53 | 6 | 0 | | | |
| 0 | 301 | 41 | 42 | | | |
| OR | 1.00 | 0.83 | 0.00 | 0.0083 | 340.8 | |
| Linear OR | 0.42 | | | 0.0083 | 343.4 | 0.5628 |
| | | | | | | |

| Result | Alleles | | | OR (95% CI) | P-value | |
|---|---|---|---|---|---|---|
| | T | C | | | | |
| 1 | 112 | 6 | | | | |
| 0 | 643 | 125 | | | | |
| Total | 755 | 131 | | 0.28 (0.12 - 0.64) | 0.003 | |
| | | | | | | |
| Comments | The C/T and C/C genotypes are linked to an increased safety, the C allele being the most important factor in explaining the safety of the treatment. | | | | | |
| | Figure 11 shows the ROC curve corresponding to the univariate model in relation to SNP rs1183035. | | | | | |

The inventors also evaluated the safety prediction capability in response to a varenicline-based pharmacological treatment, in relation to dose reduction due to adverse effects, of the set of SNPs consisting of rs763132, rs4474069 and rs1183035, with respect to the safety predictive value of each of said SNPs analyzed in an isolated manner. The inventors observed that the analysis of the set of the mentioned SNPs improves the prediction capability (AUC = 0.6978) with respect to the prediction capability of each of the SNPs previously analyzed in an individual manner (AUC (ras763132) - 0.6329; AUC (rs4474069) = 0.6078; AUC (rs1183035) = 0.5628).

**Table 6. Suspension of treatment**

| | | | | |
|---|---|---|---|---|
| OPRM1 (6q24-q25) | rs3778151 (p-0.0024)C | rs9478504 A/G | G 0,158 | INF-CG |

| Gene | ID | SNPs from which information is obtained | MAF | Function |
|---|---|---|---|---|
| | | rs590761 A/G | A 0.117 | ITR |
| | | rs653011 A/T | T 0.118 | IE |
| | | rs10485057 A/G | G 0.117 | NFI |
| | | rs632395 C/T | T 0.088 | ITR |
| | | rs645189 C/T | T 0.096 | ITR |
| | | rs650245 C/T | T 0.089 | IE-CG |
| | | rs511420 C/T | C 0.112 | NFI |
| | | rs649840 G/T | T 0.105 | IE-CG |
| | | rs616585 C/T | T 0.119 | IE-CG |
| | rs9479757 (p-.0.0002)A | rs9479756 A/G | A 0.084 | NFI |
| | | rs617648 C/T | T 0.081 | NFI |
| | | rs664247 A/G | A 0.071 | ITR |
| | | rs556716A/G | A 0.117 | IE |
| | | rs625109C/T | C 0.117 | IE |
| OPRM1 (6q24-q25) | | rs628618C/T | C 0.117 | IE-CG |
| | | rs636433C/T | T 0.117 | IE |
| | | rs544093G/T | G 0.117 | NFI |
| | | rs613341A/G | A 0.108 | IE |
| | | rs620496A/C | C 0.117 | IE |
| | | rs599945G/T | G 0.088 | NFDS-CG |
| | | **rs9479757A/G** | A 0.084 | ITR |
| | | rs499796C/T | C 0.168 | ITR |
| | | rs553202A/G | A 0.204 | ITR |
| | | rs607759C/T | T 0.175 | ITR-CG |
| | | rs520321C/T | C 0.155 | ITR |
| | rs477292 (p-0.0229)A | rs514980A/G | G 0.161 | ITR-CG |
| | | rs557748C/T | T 0.164 | ITR-CG |
| | | rs524731A/C | A 0.164 | ITR |
| | | rs511435A/G | A 0.164 | ITR |
| | | rs509544A/G | G 0.164 | ITR-CG |
| | | rs668394G/T | G 0.164 | ITR |
| | | rs634479C/T | C 0.178 | ITR |
| | | **rs477292A/G** | A 0.192 | NFI |
| | rs495491 (p-0.0031)C | No HAPMAP | C 0.181 | ITR |
| | | rs9478504 A/G | G 0.158 | NFI-CG |
| | | rs1461773C/T | T 0.136 | ITR |
| | | rs17275521A/G | A 0.144 | ITR |
| | | rs3778153 A/C | A 0.164 | ITR |
| | | rs9285542 C/T | T 0.142 | NFI |
| | | rs3778149 C/G | G 0.138 | ITR |
| | | rs3778150 C/T | C 0.137 | ITR |
| | | rs9478503 C/T | C 0.151 | ITR |
| | | rs9478501 C/T | C 0.136 | ITR |
| | | rs3778146 C/T | C 0.137 | ITR |
| | | rs6927269 G/T | G 0.137 | ITR |
| | | rs3778148 G/T | T 0.142 | ITR |
| | | rs3823010 A/G | A 0.142 | ITR |
| OPRM1 (6q24-q25) | rs3778151 (p-0.0024)C | rs17209711 A/T | A 0.146 | ITR |
| | | rs3778156 A/G | G 0.142 | IE |
| | | rs7773995 C/T | T 0.138 | IE |
| | | rs3778145 A/C | C 0.137 | ITR |
| | | rs3778158 A/T | T 0.15 | ITR |
| | | rs3778147 A/G | A 0.142 | ITR-CG |
| | | rs3778157 C/T | C 0.15 | ITR |
| | | rs9479754 C/T | C 0.138 | ITR |
| | | rs3778155 C/T | T 0.142 | ITR |
| | | rs7772959 A/G | A 0.118 | NFI-CG |
| | | rs3798678 A/G | G 0.15 | ITR |
| | | rs9322445 A/G | G 0.15 | ITR-CG |
| | | rs9478499 A/T | A 0.136 | IE |
| | | rs1381376 A/G | A 0.15 | ITR-CG |
| | | **rs3778151 C/T** | C 0.167 | NFI-CG |
| UBASH3B or STS-1(11q24.1) | rs7930792 (p-0.0256)C | rs10790522 C/T | T 0.312 | NFI |
| | | rs2233 C/G | C 0.358 | IE |
| | | rs10790521 G/T | T 0.39 | IE |
| | | rs1000722 C/T | C/T 1 | IGRT |
| | | **rs7930792 C/G** | G 0.35 | NFI |
| | | | | |
| | | rs4763340 C/T | C 0.358 | NFUS |
| | | rs970063 A/G | A 0.35 | NFUS |
| | | rs7957232 C/T | C 0.344 | USPRR |
| | | rs4763975 A/G | G 0.319 | IE |
| | | rs1993508 C/G | C 0.35 | USPRR-CG |
| | rs12423809 (p-0.0141)C | rs4558247 A/G | A 0.35 | NFUS |
| C12orf36 | | rs2077980 C/G | G 0.333 | NFI |
| | | rs1019952 C/T | T 0.325 | NFIG |
| | | rs4763976 C/T | T 0.333 | USPRR |
| | | rs4763339 C/T | T 0.35 | NFUS-CG |
| | | rs1479112 A/C | C 0.35 | NFI |
| | | rs12425036 A/G | A 0.35 | USTR |
| | | **rs12923809 A/C** | C 0.342 | NFUS-CG |
| SLC6A4 (17q11.1-q12) | rs4251417 (p-0.0149)A | **rs4251417 A/G** | A 0.111 | NFI-CG |
| C19orf6 | rs7146 (p-0.0162)T | rs2240170 C/G | C 0.333 | ITR-CG |
| | | **rs7146 C/T** | T 0.314 | SEAR -CG |

| | | | | |
|---|---|---|---|---|
| [NFUS: non-functional upstream; USPRR: Upstream promoter region regulator; USTR: Upstream transcriptional regulator; NFDS: Non-functional downstream; CG: CpG site; SEAR: Synonymous exonic assembly regulator; IGTR: Intergenic transcriptional regulator; NFIG: non-functional intergenic; NFI: non-functional intronic; IE: intronic enhancer; ITR: Intronic transcriptional regulator; ND; data not available] | | | | |

**Table 7. Dose reduction**

| Gene | ID | SNPs from which information is obtained | MAF | Function |
|---|---|---|---|---|
| ARHGAP18 (6q22-q24) | rs763132 (p-0.0007)T | rs763132 G/T | G 0.372 | IE |
| MAOB(Xp11.23) | rs1183035 (p-0.0083)T | rs1183035C/T | C 0.15 | IT ND |
| | | | | |
| | | rs7047157C/T | C 0.128 | IE-CG |
| | | rs4740175A/G | G 0.124 | IE |
| | | rs6597516A/G | A 0.127 | NFI |
| | | rs3857983A/C | A 0.125 | IE-CG |
| | | rs7853122C/T | C 0.124 | IE |
| | | rs3780261C/T | C 0.125 | IE |
| | | rs4740174C/T | T 0.119 | USPRR-CG |
| | | rs1536733A/C | A 0.125 | NFI |
| | | rs11790386C/T | T 0.121 | NFI |
| | | rs4740177C/T | T 0.125 | NFI |
| | rs4474069 (p-0.0029)C | rs7035496A/G | G 0.119 | IE |
| RAPGEF1 (9q34.3) | | rs2076C/T | T 0.121 | IE |
| | | rs2183022A/G | A 0.125 | NFI-CG |
| | | rs4474069C/T | T 0.125 | IE-CG |
| | | rs874056C/T | T 0.125 | NFI |
| | | rs7868479A/G | A 0.125 | IE |
| | | rs1541052C/T | C 0.108 | USPRR |
| | | rs3890084C/G | C 0.117 | IE-CG |
| | | rs1541055C/T | C 0.125 | IE-CG |
| | | rs2282008C/T | C 0.117 | IE-CG |
| | | rs10901075C/T | C 0.125 | IE-CG |
| | | rs943847G/T | G 0.117 | NFI |
| | | rs1410562A/C | A 0.124 | IE |
| | | rs1544305C/T | C 0.124 | IE |
| | | rs2282006C/T | C 0.125 | NFI-CG |
| | | rs2282005C/T | T 0.117 | IE |
| | | rs2282009C/T | C 0.125 | NFI-CG |
| | | rs1410563A/G | A 0.117 | IE |
| | | rs6597518 C/T | T 0.125 | NFI |
| | | rs4474069 C/T | T 0.125 | IE-CG |

| | | | | |
|---|---|---|---|---|
| [USPRR: Upstream promoter region regulator; CG: CpG site; NFI: non-functional intronic; IE: intronic enhancer; IT: intronc transcript; ND: data not available] | | | | |

## Claims

1. A method for determining the probability that a subject will suffer adverse effects in response to a nicotinic cholinergic receptor agonist drug-based therapy, wherein said adverse effects lead to the withdrawal of said therapy or to a reduction of the usual therapeutic dose of the drug used in said therapy, said method comprises determining in a biological sample of said subject at least one allele of one or more of single-nucleotide polymorphisms (SNPs) rs9479757, rs477292, rs495491, rs3778151, rs7930792, rs12423809, rs4251417, rs7146, rs763132, rs4474069 and rs1183035, , wherein
- the presence of at least one A allele of SNP rs9479757,
- the presence of at least one A allele of SNP rs477292,
- the presence of at least one C allele of SNP rs495491,
- the presence of at least one C allele of SNP rs3778151,
- the presence of at least one C allele of SNP rs7930792,
- the presence of at least one C allele of SNP rs12423809,
- the presence of at least one A allele of SNP rs4251417,
- the presence of at least one T allele of SNP rs7146,
- the presence of at least one T allele of SNP rs763132,
- the presence of at least one C allele of SNP rs4474069, and/or
- the presence of at least one T allele of SNP rs1183035,
is indicative that said subject has a high probability of suffering adverse effects in response to said nicotinic cholinergic receptor agonist drug-based therapy that lead to the withdrawal of said therapy or to a reduction of the usual therapeutic dose of the drug used in said therapy;
or, alternatively,
- the presence of at least one G allele of SNP rs9479757,
- the presence of at least one G allele of SNP rs477292,
- the presence of at least one T allele of SNP rs495491,
- the presence of at least one T allele of SNP rs3778151,
- the presence of at least one G allele of SNP rs7930792,
- the presence of at least one A allele of SNP rs12423809,
- the presence of at least one G allele of SNP rs4251417,
- the presence of at least one C allele of SNP rs7146,
- the presence of at least one G allele of SNP rs763132,
- the presence of at least one T allele of SNP rs4474069, and/or
- the presence of at least one C allele of SNP rs1183035,
is indicative that said subject has a low probability of suffering adverse effects in response to said nicotinic cholinergic receptor agonist drug-based therapy that lead to the withdrawal of said therapy or to a reduction of the usual therapeutic dose of the drug used in said therapy.

2. The method according to claim 1, wherein
- the presence of both A alleles of SNP rs9479757,
- the presence of both A alleles of SNP rs477292,
- the presence of both C alleles of SNP rs495491,
- the presence of both C alleles of SNP rs3778151,
- the presence of both C alleles of SNP rs7930792,
- the presence of both C alleles of SNP rs12423809,
- the presence of both A alleles of SNP rs4251417,
- the presence of both T alleles of SNP rs7146,
- the presence of both T alleles of SNP rs763132, ;
- the presence of both C alleles of SNP rs4474069, and/or
- the presence of both T alleles of SNP rs1183035,
is indicative that said subject has a high probability of suffering adverse effects in response to said nicotinic cholinergic receptor agonist drug-based therapy that lead to the withdrawal of said therapy or to a reduction of the usual therapeutic dose of the drug used in said therapy;
or, alternatively, wherein
- the presence of both G alleles of SNP rs9479757,
- the presence of both G alleles of SNP rs477292,
- the presence of both T alleles of SNP rs495491,
- the presence of both T alleles of SNP rs3778151,
- the presence of both G alleles of SNP rs7930792,
- the presence of both A alleles of SNP rs12423809,
- the presence of both G alleles of SNP rs4251417,
- the presence of both C alleles of SNP rs7146,
- the presence of both G alleles of SNP rs763132,
- the presence of both T alleles of SNP rs4474069, and/or
- the presence of both C alleles of SNP rs1183035,
is indicative that said subject has a low probability of suffering adverse effects in response to said nicotinic cholinergic receptor agonist drug-based therapy that lead to the withdrawal of said therapy or to a reduction of the usual therapeutic dose of the drug used in said therapy.

3. The method according to any of claims 1 or 2, wherein said adverse effects that lead to the withdrawal of said therapy or to a reduction of the usual therapeutic dose of the drug used in said therapy are selected from the group consisting of insomnia, nocturnal agitation, drowsiness, vivid dreams, headaches, dizziness, restlessness, nervous tension, symptoms of depression, bloated feeling in the stomach, abdominal distension, meteorism, flatulence, nausea, vomiting, diarrhea, constipation, dry mouth, aphthae, skin rash, fatigue, general aches and pains and combinations thereof.

4. A method for predicting the safety of a nicotinic cholinergic receptor agonist drug-based therapy, which comprises determining in a biological sample of the subject at least one allele of one or more of single-nucleotide polymorphisms (SNPs) rs9479757, rs477292, rs495491, rs3778151, rs7930792, rs12423809, rs4251417, rs7146, rs763132, rs4474069 or rs1183035, wherein
- the presence of at least one G allele of SNP rs9479757,
- the presence of at least one G allele of SNP rs477292,
- the presence of at least one T allele of SNP rs495491,
- the presence of at least one T allele of SNP rs3778151,
- the presence of at least one G allele of SNP rs7930792,
- the presence of at least one A allele of SNP rs12423809,
- the presence of at least one G allele of SNP rs4251417,
- the presence of at least one C allele of SNP rs7146,
- the presence of at least one G allele of SNP rs763132,
- the presence of at least one T allele of SNP rs4474069, and/or
- the presence of at least one C allele of SNP rs1183035,
is indicative that said nicotinic cholinergic receptor agonist drug-based therapy is safe at the usual therapeutic dose of the drug used in said therapy;
or, alternatively, wherein
- the presence of at least one A allele of SNP rs9479757,
- the presence of at least one A allele of SNP rs477292,
- the presence of at least one C allele of SNP rs495491,
- the presence of at least one C allele of SNP rs3778151,
- the presence of at least one C allele of SNP rs7930792,
- the presence of at least one C allele of SNP rs12423809,
- the presence of at least one A allele of SNP rs4251417, and/or
- the presence of at least one T allele of SNP rs7146,
is indicative that said nicotinic cholinergic receptor agonist drug-based therapy is not safe at the usual therapeutic dose of the drug used in said therapy;
or, alternatively, wherein
- the presence of at least one T allele of SNP rs763132,
- the presence of at least one C allele of SNP rs4474069, and/or
- the presence of at least one T allele of SNP rs1183035,
is indicative that said nicotinic cholinergic receptor agonist drug-based therapy is safe at a dose equal to or less than 1 mg/day of the drug used in said therapy.

5. The method according to claim 4, wherein
- the presence of both G alleles of SNP rs9479757,
- the presence of both G alleles of SNP rs477292,
- the presence of both T alleles of SNP rs495491,
- the presence of both T alleles of SNP rs3778151,
- the presence of both G alleles of SNP rs7930792,
- the presence of both A alleles of SNP rs12423809,
- the presence of both G alleles of SNP rs4251417,
- the presence of both C alleles of SNP rs7146,
- the presence of both G alleles of SNP rs763132,
- the presence of both T alleles of SNP rs4474069, and/or
- the presence of both C alleles of SNP rs1183035,
is indicative that said nicotinic cholinergic receptor agonist drug-based therapy is safe at the usual therapeutic dose of the drug used in said therapy;
or, alternatively, wherein
- the presence of both A alleles of SNP rs9479757,
- the presence of both A alleles of SNP rs477292,
- the presence of both C alleles of SNP rs495491,
- the presence of both C alleles of SNP rs3778151,
- the presence of both C alleles of SNP rs7930792,
- the presence of both C alleles of SNP rs12423809,
- the presence of both A alleles of SNP rs4251417, and/or
- the presence of both T alleles of SNP rs7146,
is indicative that said nicotinic cholinergic receptor agonist drug-based therapy is not safe at the usual therapeutic dose of the drug used in said therapy;
or alternatively, wherein
- the presence of both T alleles of SNP rs763132,
- the presence of both C alleles of SNP rs4474069, and/or
- the presence of both T alleles of SNP rs1183035,
is indicative that said nicotinic cholinergic receptor agonist drug-based therapy is safe at a dose equal to or less than 1 mg/day of the drug used in said therapy.

6. A method for selecting a subject to follow a nicotinic cholinergic receptor agonist drug-based therapy, which comprises determining in a biological sample of the subject at least one allele of one or more of single-nucleotide polymorphisms (SNPs) rs9479757, rs477292, rs495491, rs3778151, rs7930792, rs12423809, rs4251417, rs7146, rs763132, rs4474069 or rs1183035, wherein
the subject is selected for the therapy if he/she has
- at least one G allele of SNP rs9479757,
- at least one G allele of SNP rs477292,
- at least one T allele of SNP rs495491,
- at least one T allele of SNP rs3778151,
- at least one G allele of SNP rs7930792,
- at least one A allele of SNP rs12423809,
- at least one G allele of SNP rs4251417,
- at least one C allele of SNP rs7146,
- at least one G allele of SNP rs763132,
- at least one T allele of SNP rs4474069, and/or
- at least one C allele of SNP rs1183035.

7. The method according to any of claims 1 to 6, wherein said SNP is selected from the group consisting of SNPs rs9479757, rs477292, rs495491, rs3778151, rs7930792, rs12423809, rs4251417, rs7146, and any combination of said SNPs, or, alternatively, from the group consisting of SNPs rs763132, rs4474069 or rs1183035 and any combination of said SNPs.

8. A method for selecting a therapy for a subject in need of therapy which comprises determining in a biological sample of said subject at least one allele of one or more of single-nucleotide polymorphisms (SNPs) rs9479757, rs477292, rs495491, rs3778151, rs7930792, rs12423809, rs4251417, rs7146, rs763132, rs4474069 or rs1183035, wherein
a nicotinic cholinergic receptor agonist drug-based therapy is selected if said subject has
- at least one G allele of SNP rs9479757,
- at least one G allele of SNP rs477292,
- at least one T allele of SNP rs495491,
- at least one T allele of SNP rs3778151,
- at least one G allele of SNP rs7930792,
- at least one A allele of SNP rs12423809,
- at least one G allele of SNP rs4251417,
- at least one C allele of SNP rs7146,
- at least one G allele of SNP rs763132,
- at least one T allele of SNP rs4474069, and/or
- at least one C allele of SNP rs1183035.

9. The method according to any of claims 1 to 8, wherein the nicotinic cholinergic receptor agonist-based therapy is intended for the treatment of a chemical addiction or dependence.

10. The method according to claim 9, wherein said chemical addiction or dependence is a nicotine or tobacco addiction.

11. The method according to any of claims 1 to 10, wherein the subject is a tobacco consumer, preferably an active smoker.

12. A kit consisting of the reagents necessary for determining the presence of at least one allele of each of the SNPs rs9479757, rs477292, rs495491, rs3778151, rs7930792, rs12423809, rs4251417, rs7146, rs763132, rs4474069 or rs1183035, and/or the reagents for determining the nucleotide present in the polymorphic site of each one of said SNPs,
wherein the reagents necessary for determining the presence of at least one allele of each of one of said SNPs, and/or the reagents for determining the nucleotide present in the polymorphic site of said SNPs consist of:
- allele-specific or polymorphism-specific oligonucleotide pairs, where each allele-specific or polymorphism-specific oligonucleotide pair targets one of the SNPs rs9479757, rs477292, rs495491, rs3778151, rs7930792, rs12423809, rs4251417, rs7146, rs763132, rs4474069, rs1183035, or
- a set of probes, wherein said set of probes consists of a plurality of oligonucleotide probes interrogating for each of the SNPs of the group consisting of SNPs rs9479757, rs477292, rs495491, rs3778151, rs7930792, rs12423809, rs4251417, rs7146, rs763132, rs4474069, rs1183035.

13. Use of a kit according to claim 12 for:
- determining the probability that a subject will suffer adverse effects in response to a nicotinic cholinergic receptor agonist drug-based therapy, wherein said adverse effects lead to the withdrawal of said therapy or to a reduction of the usual therapeutic dose of the drug used in said therapy; or for
- predicting the safety of a nicotinic cholinergic receptor agonist drug-based therapy in a subject; or for
- selecting a subject for a nicotinic cholinergic receptor agonist drug-based therapy; or for
- selecting a therapy to be administered to a subject in need of therapy, wherein said therapy comprises a nicotinic cholinergic receptor agonist drug-based therapy.

14. Use of a single-nucleotide polymorphism (SNP) for:
- determining the probability that a subject will suffer adverse effects in response to a nicotinic cholinergic receptor agonist drug-based therapy, wherein said adverse effects lead to the withdrawal of said therapy or to a reduction of the usual therapeutic dose of the drug used in said therapy; or for
- predicting the safety of a nicotinic cholinergic receptor agonist drug-based therapy in a subject; or for
- selecting a subject for a nicotinic cholinergic receptor agonist drug-based therapy; or for
- selecting a therapy to be administered to a subject in need of therapy, wherein said therapy comprises a nicotinic cholinergic receptor agonist drug-based therapy;
wherein said SNP is selected from the group consisting of rs9479757, rs477292, rs495491, rs3778151, rs7930792, rs12423809, rs4251417, rs7146, rs763132, rs4474069 or rs1183035 and any combination thereof.

15. The method according to any of claims 1 to 11, the kit according to claim 12, use of a kit according to claim 13 or use of an SNP according to claim 14, wherein said nicotinic cholinergic receptor agonist drug is selected from the group consisting of varenicline, cytisine and dianicline.

## Patentansprüche

1. Verfahren zum Bestimmen der Wahrscheinlichkeit, dass ein Subjekt nachteilige Wirkungen in Reaktion auf eine nikotinische cholinergische Rezeptor-Agonist-Arzneimittel-basierte Therapie erleiden wird, wobei die nachteiligen Wirkungen zum Absetzen der Therapie oder zu einer Verringerung der üblichen therapeutischen Dosis des verwendeten Arzneimittels in der Therapie führen, wobei das Verfahren ein Bestimmen in wenigstens einer biologischen Probe des Subjekts wenigstens eines Alleles eines oder mehrerer einzelner Nukleotidpolymorphismen (SNPs) rs9479757, rs477292, rs495491, rs3778151, rs7930792, rs12423809, rs4251417, rs7146, rs763132, rs4474069 und rs1183035 davon umfasst, wobei
- die Anwesenheit von wenigstens einem A-Allel von SNP rs9479757,
- die Anwesenheit von wenigstens einem A-Allel von SNP rs477292,
- die Anwesenheit von wenigstens einem C-Allel von SNP rs495491,
- die Anwesenheit von wenigstens einem C-Allel von SNP rs3778151,
- die Anwesenheit von wenigstens einem C-Allel von SNP rs7930792,
- die Anwesenheit von wenigstens einem C-Allel von SNP rs12423809,
- die Anwesenheit von wenigstens einem A-Allel von SNP rs4251417,
- die Anwesenheit von wenigstens einem T-Allel von SNP rs7146,
- die Anwesenheit von wenigstens einem T-Allel von SNP rs763132,
- die Anwesenheit von wenigstens einem C-Allel von SNP rs4474069, und/oder
- die Anwesenheit von wenigstens einem T-Allel von SNP rs1183035,
anzeigt, dass das Subjekt eine hohe Wahrscheinlichkeit aufweist, nachteilige Wirkungen in Reaktion auf die nikotinische cholinerge Rezeptor-Agonist-Arzneimittel-basierte Therapie zu erleiden, die zum Absetzen der Therapie oder zu einer Verringerung der üblichen therapeutischen Dosis des in der Therapie verwendeten Arzneimittels führen; oder alternativ,
- die Anwesenheit von wenigstens einem G-Allel von SNP rs9479757,
- die Anwesenheit von wenigstens einem G-Allel von SNP rs477292,
- die Anwesenheit von wenigstens einem T-Allel von SNP rs495491,
- die Anwesenheit von wenigstens einem T-Allel von SNP rs3778151,
- die Anwesenheit von wenigstens einem G-Allel von SNP rs7930792,
- die Anwesenheit von wenigstens einem A-Allel von SNP rs12423809,
- die Anwesenheit von wenigstens einem G-Allel von SNP rs4251417,
- die Anwesenheit von wenigstens einem C-Allel von SNP rs7146,
- die Anwesenheit von wenigstens einem G-Allel von SNP rs763132,
- die Anwesenheit von wenigstens einem T-Allel von SNP rs4474069, und/oder
- die Anwesenheit von wenigstens einem C-Allel von SNP rs1183035,
anzeigt, dass das Subjekt eine geringe Wahrscheinlichkeit aufweist, nachteilige Wirkungen in Reaktion auf die nikotinische cholinerge Rezeptor-Agonist-Arzneimittel-basierte Therapie zu erleiden, die zum Absetzen der Therapie oder zu einer Verringerung der üblichen therapeutischen Dosis des in der Therapie verwendeten Arzneimittels führen.

2. Verfahren nach Anspruch 1, wobei
- die Anwesenheit von beiden A-Allelen von SNP rs9479757,
- die Anwesenheit von beiden A-Allelen von SNP rs477292,
- die Anwesenheit von beiden C-Allelen von SNP rs495491,
- die Anwesenheit von beiden C-Allelen von SNP rs3778151,
- die Anwesenheit von beiden C-Allelen von SNP rs7930792,
- die Anwesenheit von beiden C-Allelen von SNP rs12423809,
- die Anwesenheit von beiden A-Allelen von SNP rs4251417,
- die Anwesenheit von beiden T-Allelen von SNP rs7146,
- die Anwesenheit von beiden T-Allelen von SNP rs763132, ;
- die Anwesenheit von beiden C-Allelen von SNP rs4474069, und/oder
- die Anwesenheit von beiden T-Allelen von SNP rs1183035,
anzeigt, dass das Subjekt eine hohe Wahrscheinlichkeit aufweist, nachteilige Wirkungen in Reaktion auf die nikotinische cholinerge Rezeptor-Agonist-Arzneimittel-basierte Therapie zu erleiden, die zum Absetzen der Therapie oder zu einer Verringerung der üblichen therapeutischen Dosis des in der Therapie verwendeten Arzneimittels führen;
oder alternativ, wobei
- die Anwesenheit von beiden G-Allelen von SNP rs9479757,
- die Anwesenheit von beiden G-Allelen von SNP rs477292,
- die Anwesenheit von beiden T-Allelen von SNP rs495491,
- die Anwesenheit von beiden T-Allelen von SNP rs3778151,
- die Anwesenheit von beiden G-Allelen von SNP rs7930792,
- die Anwesenheit von beiden A-Allelen von SNP rs12423809,
- die Anwesenheit von beiden G-Allelen von SNP rs4251417,
- die Anwesenheit von beiden C-Allelen von SNP rs7146,
- die Anwesenheit von beiden G-Allelen von SNP rs763132,
- die Anwesenheit von beiden T-Allelen von SNP rs4474069, und/oder
- die Anwesenheit von beiden C-Allelen von SNP rs1183035,
anzeigt, dass das Subjekt eine geringe Wahrscheinlichkeit aufweist, nachteilige Wirkungen in Reaktion auf die nikotinische cholinerge Rezeptor-Agonist-Arzneimittel-basierte Therapie zu erleiden, die zum Absetzen der Therapie oder zu einer Verringerung der üblichen therapeutischen Dosis des in der Therapie verwendeten Arzneimittels führen.

3. Verfahren nach einem der Ansprüche 1 oder 2, wobei die nachteiligen Wirkungen, die zum Absetzen der Therapie oder zu einer Verringerung der üblichen therapeutischen Dosis des in der Therapie verwendeten Arzneimittels führen, ausgewählt sind aus der Gruppe bestehend aus Schlaflosigkeit, nächtlicher Unruhe, Schläfrigkeit, lebhaften Träumen, Kopfschmerzen, Schwindel, Unruhe, nervöser Spannung, Depressionssymptomen, aufgeblähtes Gefühl im Magen, Bauchdehnung, Meteorismus, Blähungen, Übelkeit, Erbrechen, Durchfall, Verstopfung, trockenem Mund, Aphthen, Hautausschlag, Müdigkeit, allgemeinen Schmerzen und Kombinationen davon.

4. Verfahren zur Vorhersage der Sicherheit einer nikotinischen cholinergen Rezeptor-Agonisten-Arzneimittel-basierten Therapie, welches ein Bestimmen in einer biologischen Probe des Patienten wenigstens eines Allels eines oder mehrerer einzelner Nukleotidpolymorphismen (SNPs) rs9479757, rs477292, rs495491, rs3778151, rs7930792, rs12423809, rs4251417, rs7146, rs763132, rs4474069 oder rs1183035 umfasst, wobei
- die Anwesenheit von wenigstens einem G-Allel von SNP rs9479757,
- die Anwesenheit von wenigstens einem G-Allel von SNP rs477292,
- die Anwesenheit von wenigstens einem T-Allel von SNP rs495491,
- die Anwesenheit von wenigstens einem T-Allel von SNP rs3778151,
- die Anwesenheit von wenigstens einem G-Allel von SNP rs7930792,
- die Anwesenheit von wenigstens einem A-Allel von SNP rs12423809,
- die Anwesenheit von wenigstens einem G-Allel von SNP rs4251417,
- die Anwesenheit von wenigstens einem C-Allel von SNP rs7146,
- die Anwesenheit von wenigstens einem G-Allel von SNP rs763132,
- die Anwesenheit von wenigstens einem T-Allel von SNP rs4474069 und/oder
- die Anwesenheit von wenigstens einem C-Allel von SNP rs1183035,
anzeigt, dass die nikotinische cholinergische Rezeptor-Agonist-Arzneimittel-basierte Therapie bei der üblichen therapeutischen Dosis des in der Therapie verwendeten Arzneimittels sicher ist;
oder alternativ, wobei
- die Anwesenheit von wenigstens einem A-Allel von SNP rs9479757,
- die Anwesenheit von wenigstens einem A-Allel von SNP rs477292,
- die Anwesenheit von wenigstens einem C-Allel von SNP rs495491,
- die Anwesenheit von wenigstens einem C-Allel von SNP rs3778151,
- die Anwesenheit von wenigstens einem C-Allel von SNP rs7930792,
- die Anwesenheit von wenigstens einem C-Allel von SNP rs12423809,
- die Anwesenheit von wenigstens einem A-Allel von SNP rs4251417, und/oder
- die Anwesenheit von wenigstens einem T-Allel von SNP rs7146,
anzeigt, dass die nikotinische cholinergische Rezeptor-Agonist-Arzneimittel-basierte Therapie bei der üblichen therapeutischen Dosis des in der Therapie verwendeten Arzneimittels nicht sicher ist;
oder alternativ, wobei
- die Anwesenheit von wenigstens einem T-Allel von SNP rs763132,
- die Anwesenheit von wenigstens einem C-Allel von SNP rs4474069 und/oder
- die Anwesenheit von wenigstens einem T-Allel von SNP rs1183035, anzeigt, dass die nikotinische cholinergische Rezeptor-Agonist-Arzneimittel-basierte Therapie bei einer Dosis gleich oder weniger als 1 mg/Tag des in der Therapie verwendeten Arzneimittels sicher ist.

5. Verfahren nach Anspruch 4, wobei
- die Anwesenheit von beiden G-Allelen von SNP rs9479757,
- die Anwesenheit von beiden G-Allelen von SNP rs477292,
- die Anwesenheit von beiden T-Allelen von SNP rs495491,
- die Anwesenheit von beiden T-Allelen von SNP rs3778151,
- die Anwesenheit von beiden G-Allelen von SNP rs7930792,
- die Anwesenheit von beiden A-Allelen von SNP rs12423809,
- die Anwesenheit von beiden G-Allelen von SNP rs4251417,
- die Anwesenheit von beiden C-Allelen von SNP rs7146,
- die Anwesenheit von beiden G-Allelen von SNP rs763132,
- die Anwesenheit von beiden T-Allelen von SNP rs4474069, und/oder
- die Anwesenheit von beiden C-Allelen von SNP rs1183035,
anzeigt, dass die nikotinische cholinergische Rezeptor-Agonist-Arzneimittel-basierte Therapie bei der üblichen therapeutischen Dosis des in der Therapie verwendeten Arzneimittels sicher ist;
oder alternativ, wobei
- die Anwesenheit von beiden A-Allelen von SNP rs9479757,
- die Anwesenheit von beiden A-Allelen von SNP rs477292,
- die Anwesenheit von beiden C-Allelen von SNP rs495491,
- die Anwesenheit von beiden C-Allelen von SNP rs3778151,
- die Anwesenheit von beiden C-Allelen von SNP rs7930792,
- die Anwesenheit von beiden C-Allelen von SNP rs12423809,
- die Anwesenheit von beiden A-Allelen von SNP rs4251417, und/oder
- die Anwesenheit von beiden T-Allelen von SNP rs7146,
anzeigt, dass die nikotinische cholinergische Rezeptor-Agonist-Arzneimittel-basierte Therapie bei der üblichen therapeutischen Dosis des in der Therapie verwendeten Arzneimittels nicht sicher ist;
oder alternativ, wobei
- die Anwesenheit von beiden T-Allelen von SNP rs763132,
- die Anwesenheit von beiden C-Allelen von SNP rs4474069, und/oder
- die Anwesenheit von beiden T-Allelen von SNP rs1183035,
anzeigt, dass die nikotinische cholinergische Rezeptor-Agonist-Arzneimittel-basierte Therapie bei einer Dosis gleich oder weniger als 1 mg/Tag des in der Therapie verwendeten Arzneimittels sicher ist.

6. Verfahren zum Auswählen eines Subjekts, um einer nikotinischen cholinergen Rezeptor-Agonisten-Arzneimittel-basierten Therapie zu folgen, welches ein Bestimmen in einer biologischen Probe des Subjekts wenigstens eines Allels eines oder mehrerer einzelner Nukleotidpolymorphismen (SNPs) rs9479757, rs477292, rs495491, rs3778151, rs7930792, rs12423809, rs4251417, rs7146, rs763132, rs4474069 oder rs1183035 umfasst, wobei das Subjekt für die Therapie ausgewählt wird, wenn er/sie
- wenigstens ein G-Allel von SNP rs9479757;
- wenigstens ein G-Allel von SNP rs477292;
- wenigstens ein T-Allel von SNP rs495491;
- wenigstens ein T-Allel von SNP rs3778151;
- wenigstens ein G-Allel von SNP rs7930792;
- wenigstens ein A-Allel von SNP rs12423809;
- wenigstens ein G-Allel von SNP rs4251417;
- wenigstens ein C-Allel von SNP rs7146;
- wenigstens ein G-Allel von SNP rs763132;
- wenigstens ein T-Allel von SNP rs4474069, und/oder
- wenigstens ein C-Allel von SNP rs1183035 aufweist.

7. Verfahren nach einem der Ansprüche 1 bis 6, wobei der SNP ausgewählt ist aus der Gruppe bestehend aus den SNPs rs9479757, rs477292, rs495491, rs3778151, rs7930792, rs12423809, rs4251417, rs7146 und einer beliebigen Kombination der SNPs oder alternativ aus der Gruppe bestehend aus den SNPs rs763132, rs4474069 oder rs1183035 und einer beliebigen Kombination der SNPs.

8. Verfahren zum Auswählen einer Therapie für ein Subjekt, das einer Therapie bedarf, welches ein Bestimmen in einer biologischen Probe des Subjekts wenigstens eines Allels eines oder mehrerer einzelner Nukleotidpolymorphismen (SNPs) rs9479757, rs477292, rs495491, rs3778151, rs7930792, rs12423809, rs4251417, rs7146, rs763132, rs4474069 oder rs1183035 umfasst, wobei
eine nikotinische cholinergische Rezeptor-Agonist-Arzneimittel-basierte Therapie ausgewählt wird, wenn das Subjekt
- wenigstens ein G-Allel von SNP rs9479757;
- wenigstens ein G-Allel von SNP rs477292;
- wenigstens ein T-Allel von SNP rs495491;
- wenigstens ein T-Allel von SNP rs3778151;
- wenigstens ein G-Allel von SNP rs7930792;
- wenigstens ein A-Allel von SNP rs12423809;
- wenigstens ein G-Allel von SNP rs4251417;
- wenigstens ein C-Allel von SNP rs7146;
- wenigstens ein G-Allel von SNP rs763132;
- wenigstens ein T-Allel von SNP rs4474069; und/oder
- wenigstens ein C-Allel von SNP rs1183035 aufweist.

9. Verfahren nach einem der Ansprüche 1 bis 8, wobei die nikotinische cholinergische Rezeptor-Agonisten-basierte Therapie zur Behandlung einer chemischen Sucht oder Abhängigkeit bestimmt ist.

10. Verfahren nach Anspruch 9, wobei die chemische Sucht oder Abhängigkeit eine Nikotin- oder Tabaksucht ist.

11. Verfahren nach einem der Ansprüche 1 bis 10, wobei das Subjekt ein Tabakkonsument, vorzugsweise ein aktiver Raucher ist.

12. Kit bestehend aus den Reagenzien, die zum Bestimmen das Vorliegen wenigstens eines Allels eines jeden der SNPs rs9479757, rs477292, rs495491, rs3778151, rs7930792, rs12423809, rs4251417, rs7146, rs763132, rs4474069 oder rs1183035, und/oder das Vorliegen des an der polymorphen Stelle eines jeden der SNPs vorliegenden Nukleotids, notwendig sind,
wobei die Reagenzien, die zum Bestimmen des Vorliegens wenigstens eines Allels eines jeden der SNPs und/oder zum Bestimmen des an der polymorphen Stelle des SNPs vorliegenden Nukleotids notwendig sind, das bestehen aus:
- allelspezifischen oder polymorphismussspezifischen Oligonukleotidpaaren, wobei jedes allelspezifische oder polymorphismusspezifische Oligonukleotidpaar auf einen der SNPs rs9479757, rs477292, rs495491, rs3778151, rs7930792, rs12423809, rs4251417, rs7146, rs763132, rs4474069, rs1183035 abzielt, oder
- einem Satz von Sonden, wobei der Satz von Sonden aus einer Vielzahl von Oligonukleotidsonden besteht, die jeden der SNPs der Gruppe bestehend aus den SNPs rs9479757, rs477292, rs495491, rs3778151, rs7930792, rs12423809, rs4251417, rs7146, rs763132, rs4474069, rs1183035 abfragen.

13. Verwendung eines Kits nach Anspruch 12 zum:
- Bestimmen der Wahrscheinlichkeit, dass ein Subjekt nachteilige Wirkungen in Reaktion auf eine nikotinische cholinergische Rezeptor-Agonist-Arzneimittel-basierte Therapie erleiden wird, wobei die nachteiligen Wirkungen zum Absetzen der Therapie oder zu einer Verringerung der üblichen therapeutischen Dosis des in der Therapie verwendeten Arzneimittels führen; oder zum
- Vorhersagen der Sicherheit einer nikotinischen cholinergischen Rezeptor-Agonisten-Arzneimittel-Therapie in einem Subjekt; oder zum
- Auswählen eines Subjekts für eine nikotinische cholinergische Rezeptor-Agonist-Arzneimittel-basierte Therapie; oder zum
- Auswählen einer Therapie, die an ein Subjekt verabreicht werden soll, das einer Therapie bedarf, wobei das Verfahren eine nikotinische cholinergische Rezeptor-Agonist-Arzneimittel-basierte Therapie umfasst.

14. Verwendung eines Einzelnukleotid-Polymorphismus (SNP) zum:
- Bestimmen der Wahrscheinlichkeit, dass ein Subjekt nachteilige Wirkungen in Reaktion auf eine nikotinische cholinerge Rezeptor-Agonist-Arzneimittel-basierte Therapie erleiden wird, wobei die nachteiligen Wirkungen zum Absetzen der Therapie oder zu einer Verringerung der üblichen therapeutischen Dosis des in der Therapie verwendeten Arzneimittels führen; oder zum
- Vorhersagen der Sicherheit einer nikotinischen cholinergischen Rezeptor-Agonisten-Arzneimittel-Therapie in einem Subjekt; oder zum
- Auswählen eines Subjekts für eine nikotinische cholinergische Rezeptor-Agonist-Arzneimittel-basierte Therapie; oder zum
- Auswählen einer Therapie, die an ein Subjekt verabreicht werden soll, das einer Therapie bedarf, wobei das Verfahren eine nikotinische cholinergische Rezeptor-Agonist-Arzneimittel-basierte Therapie umfasst
wobei der SNP ausgewählt ist aus der Gruppe bestehend aus rs9479757, rs477292, rs495491, rs3778151, rs7930792, rs12423809, rs4251417, rs7146, rs763132, rs4474069 oder rs1183035 und einer beliebigen Kombination davon.

15. Verfahren nach einem der Ansprüche 1 bis 11, Kit nach Anspruch 12, Verwendung eines Kits nach Anspruch 13 oder Verwendung eines SNP nach Anspruch 14, wobei das nikotinische cholinergische Rezeptor-Agonist-Arzneimittel ausgewählt ist aus der Gruppe bestehend aus Vareniclin, Cytisin und Dianiclin.

## Revendications

1. Procédé pour déterminer la probabilité qu'un sujet souffrira d'effets indésirables en réponse à un traitement à base d'un médicament agoniste des récepteurs cholinergiques nicotiniques, dans lequel lesdits effets indésirables mènent à l'arrêt dudit traitement ou à une réduction de la dose thérapeutique habituelle du médicament utilisé dans ledit traitement, ledit procédé comprend la détermination dans un échantillon biologique dudit sujet d'au moins un allèle d'un ou de plusieurs polymorphismes d'un seul nucléotide (SNP) rs9479757, rs477292, rs495491, rs3778151, rs7930792, rs12423809, rs4251417, rs7146, rs763132, rs4474069 et rs1183035, , dans lequel
- la présence d'au moins un allèle A du SNP rs9479757,
- la présence d'au moins un allèle A du SNP rs477292,
- la présence d'au moins un allèle C du SNP rs495491,
- la présence d'au moins un allèle C du SNP rs3778151,
- la présence d'au moins un allèle C du SNP rs7930792,
- la présence d'au moins un allèle C du SNP rs12423809,
- la présence d'au moins un allèle A du SNP rs4251417,
- la présence d'au moins un allèle T du SNP rs7146,
- la présence d'au moins un allèle T du SNP rs763132,
- la présence d'au moins un allèle C du SNP rs4474069, et/ou
- la présence d'au moins un allèle T du SNP rs1183035,
indique que ledit sujet présente une probabilité élevée de souffrir d'effets indésirables en réponse au dit traitement à base d'un médicament agoniste des récepteurs cholinergiques nicotiniques qui mènent à l'arrêt dudit traitement ou à une réduction de la dose thérapeutique habituelle du médicament utilisé dans ledit traitement;
ou, alternativement,
- la présence d'au moins un allèle G du SNP rs9479757,
- la présence d'au moins un allèle G du SNP rs477292,
- la présence d'au moins un allèle T du SNP rs495491,
- la présence d'au moins un allèle T du SNP rs3778151,
- la présence d'au moins un allèle G du SNP rs7930792,
- la présence d'au moins un allèle A du SNP rs12423809,
- la présence d'au moins un allèle G du SNP rs4251417,
- la présence d'au moins un allèle C du SNP rs7146,
- la présence d'au moins un allèle G du SNP rs763132,
- la présence d'au moins un allèle T du SNP rs4474069, et/ou
- la présence d'au moins un allèle C du SNP rs1183035,
indique que ledit sujet présente une probabilité faible de souffrir d'effets indésirables en réponse au dit traitement à base d'un médicament agoniste des récepteurs cholinergiques nicotiniques qui mènent à l'arrêt dudit traitement ou à une réduction de la dose thérapeutique habituelle du médicament utilisé dans ledit traitement.

2. Procédé selon la revendication 1, dans lequel
- la présence des deux allèles A du SNP rs9479757,
- la présence des deux allèles A du SNP rs477292,
- la présence des deux allèles C du SNP rs495491,
- la présence des deux allèles C du SNP rs3778151,
- la présence des deux allèles C du SNP rs7930792,
- la présence des deux allèles C du SNP rs12423809,
- la présence des deux allèles A du SNP rs4251417,
- la présence des deux allèles T du SNP rs7146,
- la présence des deux allèles T du SNP rs763132,
- la présence des deux allèles C du SNP rs4474069, et/ou
- la présence des deux allèles T du SNP rs1183035,
indique que ledit sujet présente une probabilité élevée de souffrir d'effets indésirables en réponse au dit traitement à base d'un médicament agoniste des récepteurs cholinergiques nicotiniques qui mènent à l'arrêt dudit traitement ou à une réduction de la dose thérapeutique habituelle du médicament utilisé dans ledit traitement;
ou, alternativement, dans lequel
- la présence des deux allèles G du SNP rs9479757,
- la présence des deux allèles G du SNP rs477292,
- la présence des deux allèles T du SNP rs495491,
- la présence des deux allèles T du SNP rs3778151,
- la présence des deux allèles G du SNP rs7930792,
- la présence des deux allèles A du SNP rs12423809,
- la présence des deux allèles G du SNP rs4251417,
- la présence des deux allèles C du SNP rs7146,
- la présence des deux allèles G du SNP rs763132,
- la présence des deux allèles T du SNP rs4474069, et/ou
- la présence des deux allèles C du SNP rs1183035,
indique que ledit sujet présente une probabilité faible de souffrir d'effets indésirables en réponse au dit traitement à base d'un médicament agoniste des récepteurs cholinergiques nicotiniques qui mènent à l'arrêt dudit traitement ou à une réduction de la dose thérapeutique habituelle du médicament utilisé dans ledit traitement.

3. Procédé selon l'une quelconque des revendications 1 ou 2, dans lequel lesdits effets indésirables qui mènent à l'arrêt dudit traitement ou à une réduction de la dose thérapeutique habituelle du médicament utilisé dans ledit traitement sont choisis dans le groupe constitué de l'insomnie, l'agitation nocturne, la somnolence, les rêves d'apparence réelle, les céphalées, les vertiges, la nervosité, la tension nerveuse, les symptômes de dépression, la sensation de ballonnement dans l'estomac, la distension abdominale, le météorisme, les flatulences, les nausées, les vomissements, les diarrhées, la constipation, la sécheresse buccale, les aphtes, les éruptions cutanées, la fatigue, les courbatures et les douleurs générales et leurs combinaisons.

4. Procédé pour prédire l'innocuité d'un traitement à base d'un médicament agoniste des récepteurs cholinergiques nicotiniques, qui comprend la détermination dans un échantillon biologique du sujet d'au moins un allèle d'un ou de plusieurs polymorphismes d'un seul nucléotide (SNP) rs9479757, rs477292, rs495491, rs3778151, rs7930792, rs12423809, rs4251417, rs7146, rs763132, rs4474069 ou rs1183035, dans lequel
- la présence d'au moins un allèle G du SNP rs9479757,
- la présence d'au moins un allèle G du SNP rs477292,
- la présence d'au moins un allèle T du SNP rs495491,
- la présence d'au moins un allèle T du SNP rs3778151,
- la présence d'au moins un allèle G du SNP rs7930792,
- la présence d'au moins un allèle A du SNP rs12423809,
- la présence d'au moins un allèle G du SNP rs4251417,
- la présence d'au moins un allèle C du SNP rs7146,
- la présence d'au moins un allèle G du SNP rs763132,
- la présence d'au moins un allèle T du SNP rs4474069, et/ou
- la présence d'au moins un allèle C du SNP rs1183035,
indique que ledit traitement à base d'un médicament agoniste des récepteurs cholinergiques nicotiniques est sans danger à la dose thérapeutique habituelle du médicament utilisé dans ledit traitement;
ou, alternativement, dans lequel
- la présence d'au moins un allèle A du SNP rs9479757,
- la présence d'au moins un allèle A du SNP rs477292,
- la présence d'au moins un allèle C du SNP rs495491,
- la présence d'au moins un allèle C du SNP rs3778151,
- la présence d'au moins un allèle C du SNP rs7930792,
- la présence d'au moins un allèle C du SNP rs12423809,
- la présence d'au moins un allèle A du SNP rs4251417, et/ou
- la présence d'au moins un allèle T du SNP rs7146,
indique que ledit traitement à base d'un médicament agoniste des récepteurs cholinergiques nicotiniques n'est pas sans danger à la dose thérapeutique habituelle du médicament utilisé dans ledit traitement ;
ou, alternativement, dans lequel
- la présence d'au moins un allèle T du SNP rs763132,
- la présence d'au moins un allèle C du SNP rs4474069, et/ou
- la présence d'au moins un allèle T du SNP rs1183035,
indique que ledit traitement à base d'un médicament agoniste des récepteurs cholinergiques nicotiniques est sans danger à une dose inférieure ou égale à 1 mg/jour du médicament utilisé dans ledit traitement.

5. Procédé selon la revendication 4, dans lequel
- la présence des deux allèles G du SNP rs9479757,
- la présence des deux allèles G du SNP rs477292,
- la présence des deux allèles T du SNP rs495491,
- la présence des deux allèles T du SNP rs3778151,
- la présence des deux allèles G du SNP rs7930792,
- la présence des deux allèles A du SNP rs12423809,
- la présence des deux allèles G du SNP rs4251417,
- la présence des deux allèles C du SNP rs7146,
- la présence des deux allèles G du SNP rs763132,
- la présence des deux allèles T du SNP rs4474069, et/ou
- la présence des deux allèles C du SNP rs1183035,
indique que ledit traitement à base d'un médicament agoniste des récepteurs cholinergiques nicotiniques est sans danger à la dose thérapeutique habituelle du médicament utilisé dans ledit traitement ;
ou, alternativement, dans lequel
- la présence des deux allèles A du SNP rs9479757,
- la présence des deux allèles A du SNP rs477292,
- la présence des deux allèles C du SNP rs495491,
- la présence des deux allèles C du SNP rs3778151,
- la présence des deux allèles C du SNP rs7930792,
- la présence des deux allèles C du SNP rs12423809,
- la présence des deux allèles A du SNP rs4251417, et/ou
- la présence des deux allèles T du SNP rs7146,
indique que ledit traitement à base d'un médicament agoniste des récepteurs cholinergiques nicotiniques n'est pas sans danger à la dose thérapeutique habituelle du médicament utilisé dans ledit traitement ;
ou, alternativement, dans lequel
- la présence des deux allèles T du SNP rs763132,
- la présence des deux allèles C du SNP rs4474069, et/ou
- la présence des deux allèles T du SNP rs1183035,
indique que ledit traitement à base d'un médicament agoniste des récepteurs cholinergiques nicotiniques est sans danger à une dose inférieure ou égale à 1 mg/jour du médicament utilisé dans ledit traitement.

6. Procédé pour choisir un sujet pour suivre un traitement à base d'un médicament agoniste des récepteurs cholinergiques nicotiniques, qui comprend la détermination dans un échantillon biologique du sujet d'au moins un allèle d'un ou de plusieurs polymorphismes d'un seul nucléotide (SNP) rs9479757, rs477292, rs495491, rs3778151, rs7930792, rs12423809, rs4251417, rs7146, rs763132, rs4474069 ou rs1183035, dans lequel
le sujet est choisi pour le traitement s'il/si elle présente
- au moins un allèle G du SNP rs9479757,
- au moins un allèle G du SNP rs477292,
- au moins un allèle T du SNP rs495491,
- au moins un allèle T du SNP rs3778151,
- au moins un allèle G du SNP rs7930792,
- au moins un allèle A du SNP rs12423809,
- au moins un allèle G du SNP rs4251417,
- au moins un allèle C du SNP rs7146,
- au moins un allèle G du SNP rs763132,
- au moins un allèle T du SNP rs4474069, et/ou
- au moins un allèle C du SNP rs1183035.

7. Procédé selon l'une quelconque des revendications 1 à 6, dans lequel ledit SNP est choisi dans le groupe constitué des SNP rs9479757, rs477292, rs495491, rs3778151, rs7930792, rs12423809, rs4251417, rs7146, et de toute combinaison desdits SNP, ou, alternativement, dans le groupe constitué des SNP rs763132, rs4474069 ou rs1183035 et de toute combinaison desdits SNP.

8. Procédé pour choisir un traitement pour un sujet ayant besoin d'un traitement qui comprend la détermination dans un échantillon biologique dudit sujet d'au moins un allèle d'un ou de plusieurs polymorphismes d'un seul nucléotide (SNP) rs9479757, rs477292, rs495491, rs3778151, rs7930792, rs12423809, rs4251417, rs7146, rs763132, rs4474069 ou rs1183035, dans lequel
un traitement à base d'un médicament agoniste des récepteurs cholinergiques nicotiniques est choisi si ledit sujet présente
- au moins un allèle G du SNP rs9479757,
- au moins un allèle G du SNP rs477292,
- au moins un allèle T du SNP rs495491,
- au moins un allèle T du SNP rs3778151,
- au moins un allèle G du SNP rs7930792,
- au moins un allèle A du SNP rs12423809,
- au moins un allèle G du SNP rs4251417,
- au moins un allèle C du SNP rs7146,
- au moins un allèle G du SNP rs763132,
- au moins un allèle T du SNP rs4474069, et/ou
- au moins un allèle C du SNP rs1183035.

9. Procédé selon l'une quelconque des revendications 1 à 8, dans lequel le traitement à base d'un agoniste des récepteurs cholinergiques nicotiniques est prévu pour le traitement d'une accoutumance ou d'une dépendance à des substances chimiques.

10. Procédé selon la revendication 9, dans lequel ladite accoutumance ou dépendance à des substances chimiques est une accoutumance à la nicotine ou au tabac.

11. Procédé selon l'une quelconque des revendications 1 à 10, dans lequel le sujet est un consommateur de tabac, de préférence un fumeur actif.

12. Kit constitué des réactifs nécessaires pour déterminer la présence d'au moins un allèle de chacun des SNP rs9479757, rs477292, rs495491, rs3778151, rs7930792, rs12423809, rs4251417, rs7146, rs763132, rs4474069 ou rs1183035, et/ou les réactifs pour déterminer le nucléotide présent dans le site polymorphe de chacun desdits SNP,
dans lequel les réactifs nécessaires pour déterminer la présence d'au moins un allèle de chacun desdits SNP, et/ou les réactifs pour déterminer le nucléotide présent dans le site polymorphe desdits SNP sont constitués de :
- paires d'oligonucléotides spécifiques de l'allèle ou spécifiques du polymorphisme, où chaque paire d'oligonucléotides spécifiques de l'allèle ou spécifiques du polymorphisme cible l'un des SNP rs9479757, rs477292, rs495491, rs3778151, rs7930792, rs12423809, rs4251417, rs7146, rs763132, rs4474069 ou rs1183035, ou
- un ensemble de sondes, dans lequel ledit ensemble de sondes est constitué d'une pluralité de sondes oligonucléotidiques interrogeant pour chacun des SNP du groupe constitué des SNP rs9479757, rs477292, rs495491, rs3778151, rs7930792, rs12423809, rs4251417, rs7146, rs763132, rs4474069, rs1183035.

13. Utilisation d'un kit selon la revendication 12 pour:
- déterminer la probabilité qu'un sujet souffre d'effets indésirables en réponse à un traitement à base d'un médicament agoniste des récepteurs cholinergiques nicotiniques, dans lequel lesdits effets indésirables mènent à l'arrêt dudit traitement ou à une réduction de la dose thérapeutique habituelle du médicament utilisé dans ledit traitement; ou pour
- prédire l'innocuité d'un traitement à base d'un médicament agoniste des récepteurs cholinergiques nicotiniques chez un sujet; ou pour
- choisir un sujet pour un traitement à base d'un médicament agoniste des récepteurs cholinergiques nicotiniques; ou pour
- choisir un traitement à administrer à un sujet ayant besoin d'un traitement, dans lequel ledit traitement comprend un traitement à base d'un médicament agoniste des récepteurs cholinergiques nicotiniques.

14. Utilisation d'un polymorphisme d'un seul nucléotide (SNP) pour :
- déterminer la probabilité qu'un sujet souffre d'effets indésirables en réponse à un traitement à base d'un médicament agoniste des récepteurs cholinergiques nicotiniques, dans laquelle lesdits effets indésirables mènent à l'arrêt dudit traitement ou à une réduction de la dose thérapeutique habituelle du médicament utilisé dans ledit traitement ; ou pour
- prédire l'innocuité d'un traitement à base d'un médicament agoniste des récepteurs cholinergiques nicotiniques chez un sujet; ou pour
- choisir un sujet pour un traitement à base d'un médicament agoniste des récepteurs cholinergiques nicotiniques; ou pour
- choisir un traitement à administrer à un sujet ayant besoin d'un traitement, dans laquelle ledit traitement comprend un traitement à base d'un médicament agoniste des récepteurs cholinergiques nicotiniques;
dans laquelle ledit SNP est choisi dans le groupe constitué de rs9479757, rs477292, rs495491, rs3778151, rs7930792, rs12423809, rs4251417, rs7146, rs763132, rs4474069 ou rs1183035 et de toutes leurs combinaisons.

15. Procédé selon l'une quelconque des revendications 1 à 11, kit selon la revendication 12, utilisation d'un kit selon la revendication 13 ou utilisation d'un SNP selon la revendication 14, dans lequel ledit médicament agoniste des récepteurs cholinergiques nicotiniques est choisi dans le groupe constitué de la varénicline, la cytisine et la dianicline.


### SEQUENCE LISTING

<110> Genetracer Biotech S.L.
   Universidad del País Vasco
<120> MÉTODO PARA PREDECIR LA SEGURIDAD DE UN TRATAMIENTO FARMACOLÓGICO
<130> P7904PC00
<150> P201230590
   <151> 2012-04-20
<160> 11
<170> Patent In version 3.5
<210> 1
   <211> 52
   <212> DNA
   <213> Homo sapiens
<400> 1
   tcttgtttca gaaaataagg tcccagsaaa gtcaatgggt taggaagtgt gg 52
<210> 2
   <211> 52
   <212> DNA
   <213> Homo sapiens
<400> 2
   aggagaaaga tgaatatccc agctcamgca gccaacaaat ttgcccttcc tc 52
<210> 3
   <211> 52
   <212> DNA
   <213> Homo sapiens
<400> 3
   acccctgagg actcctgaga acacacrttg ccctcaaaaa tctacctacc tg 52
<210> 4
   <211> 52
   <212> DNA
   <213> Homo sapiens
<400> 4
   cgctcgccca tcaactgcct ggagcaygtg cgtgacaagt ggccgcgtga gg 52
<210> 5
   <211> 52
   <212> DNA
   <213> Homo sapiens
<400> 5
   tcagctaaat agacatactt gcttcartcc ccagatgctt tccctgtcgc tt 52
<210> 6
   <211> 52
   <212> DNA
   <213> Homo sapiens
<400> 6
   tgataggcac tggttctaca gtgagayata tctctcctaa gtctggtgac aa 52
<210> 7
   <211> 52
   <212> DNA
   <213> Homo sapiens
<400> 7
   tcaagatagc taattgagaa caagcaygag actccactcc tggtccccaa gc 52
<210> 8
   <211> 52
   <212> DNA
   <213> Homo sapiens
<400> 8
   gtgatgttac cagcctgagg gaaggarggt tcacagcctg atatgttggt ga 52
<210> 9
   <211> 52
   <212> DNA
   <213> Homo sapiens
<400> 9
   aaggtggaaa atgacataca ttcttgkgac tttagatgaa tcagattata gc 52
<210> 10
   <211> 52
   <212> DNA
   <213> Homo sapiens
<400> 10
   tctcactgtc tgtaatgact ccagcaygaa aagctcccag ccacaggcag ct 52
<210> 11
   <211> 52
   <212> DNA
   <213> Homo sapiens
<400> 11
   tcactgtaga actagaaaaa tggtcaytta ttgcaaagtg ctacagtgaa aa 52